# EUROPEAN PATENT SPECIFICATION

(11) **EP 4 502 934 B1**
(45) Date of publication and mention of the grant of the patent: **04.11.2026**
(21) Application number: 23858857.8
(22) Date of filing: 27.06.2023
(51) Int. Cl.: G06T 7/00

(54) **LESION AREA DETERMINING METHOD AND APPARATUS, AND MODEL TRAINING METHOD AND APPARATUS**
VERFAHREN UND VORRICHTUNG ZUR BESTIMMUNG EINES LÄSIONSBEREICHS SOWIE MODELLTRAININGSVERFAHREN UND -VORRICHTUNG
PROCÉDÉ ET APPAREIL DE DÉTERMINATION DE ZONE DE LÉSION, ET PROCÉDÉ ET APPAREIL D'APPRENTISSAGE DE MODÈLE

(30) Priority: 31.08.2022 CN 202211056712
(43) Date of publication of application: 05.02.2025
(73) Proprietor: Tencent Technology (Shenzhen) Company Limited, Shenzhen, Guangdong 518057 (CN)
(72) Inventor: YANG, Zhongyi, Shenzhen, Guangdong 518057 (CN); YANG, Sen, Shenzhen, Guangdong 518057 (CN); XIANG, Jinxi, Shenzhen, Guangdong 518057 (CN); ZHANG, Jun, Shenzhen, Guangdong 518057 (CN); HAN, Xiao, Shenzhen, Guangdong 518057 (CN)
(74) Representative: Novagraaf International SA
(86) International application number: PCT/CN2023/102592
(87) International publication number: WO 2024/045819

(56) References cited:
- CN-A- 105 550 651
- CN-A- 106 682 435
- CN-A- 109 523 535
- CN-A- 109 919 928
- CN-A- 111 402 228
- CN-A- 111 738 992
- CN-B- 107 133 638
- KR-A- 20210 063 116
- "Neural Networks and Deep Learning", 1 January 2018, SPRINGER, ISBN: 978-3-319-94462-3, article AGGARWAL CHARU C.: "Pooling", pages: 326 - 327, XP093283300

## Description

### RELATED APPLICATION

This application claims priority to Chinese Patent Application No. 202211056712.6, entitled "METHOD FOR DETERMINING LESION REGION, AND MODEL TRAINING METHOD AND APPARATUS FOR PATHOLOGICAL IMAGE" filed on August 31, 2022.

### FIELD OF THE TECHNOLOGY

This application relates to the field of computer vision, and in particular, to a method for determining a lesion region, and a model training method and apparatus.

### BACKGROUND OF THE DISCLOSURE

At present, a specific lesion location can be determined from a pathological image.

In the related art, a histopathological examination begins with biopsy, where a doctor obtains tissue slices from the body of a user and makes slides through steps of embedding, staining or the like. Afterwards, a pathologist places the slides under a microscope for observation in order to find the specific lesion location from the pathological image. For example, CN 109919928 discloses a medical image detection method, which includes: obtaining a medical image and pathological text information of a to-be-detected object; secondly, performing pathological type prediction on the medical image; obtaining prediction information, through the trained multi-layer perceptron, identifying pathological text information to acquire medical information; then, fusing the prediction information and the reference information to obtain a prediction result, and when the prediction result indicates that the medical image is a target pathological type, detecting an area conforming to the target pathological type from the medical image to obtain a detection result.

However, in the above related art, the efficiency of determining a lesion location is relatively low.

### SUMMARY

Embodiments of this application provide a method for determining a lesion region, and a model training method and apparatus. The technical solutions are described as follows.

The invention is set out in the appended set of claims.

The technical solutions provided in the embodiments of this application may have the following beneficial effects:

the pathological image is sampled to obtain the instance images, the feature information is extracted from the instance images, and the lesion region in the pathological image is automatically determined based on the feature information of the instance images, thereby reducing the consumption of human resources and saving costs required to determine the lesion region.

In addition, in the embodiments of this application, firstly, the candidate lesion region in the pathological image is determined based on the first sampling scheme, then the second instance image is acquired based on the second sampling scheme with a greater sampling overlap degree than that of the first sampling scheme, and based on the second instance image, the lesion region in the pathological image is determined from the candidate lesion region. Thus the area of a region in the pathological image that needs to be sampled by the second sampling scheme is reduced, thereby reducing the number of the second instance images that need to be collected and feature information extracted, reducing computational resources required to determine the lesion region, and improving the efficiency of determining the lesion region.

Moreover, the candidate lesion region is more likely to include the lesion region than other regions, and has a relatively high signal-to-noise ratio. Thus, by executing the second sampling scheme only for the candidate lesion region, an information loss can be reduced and perception of a lesion region with a relatively small area can be enhanced, thereby determining the lesion region in the pathological image more accurately.

### BRIEF DESCRIPTION OF THE DRAWINGS

FIG. 1 is a schematic diagram of a model architecture of a lesion region determination model provided in an embodiment of this application.
FIG. 2 is a schematic diagram of a lesion region determination system provided in an embodiment of this application.
FIG. 3 is a flowchart of a method for determining a lesion region provided in an embodiment of this application.
FIG. 4 is a schematic diagram of a method for determining a lesion region provided in an embodiment of this application.
FIG. 5 is a schematic diagram of a method for determining a lesion region provided in another embodiment of this application.
FIG. 6 is a schematic diagram of a method for determining a lesion region provided in another embodiment of this application.
FIG. 7 is a schematic diagram of a method for determining a lesion region provided in another embodiment of this application.
FIG. 8 is a flowchart of a method for training a lesion region determination model provided in an embodiment of this application.
FIG. 9 is a schematic diagram of a method for training a lesion region determination model provided in an embodiment of this application.
FIG. 10 is a schematic diagram of a method for determining a lesion region provided in another embodiment of this application.
FIG. 11 is a block diagram of an apparatus for determining a lesion region provided in an embodiment of this application.
FIG. 12 is a block diagram of an apparatus for determining a lesion region provided in another embodiment of this application.
FIG. 13 is a block diagram of an apparatus for training a lesion region determination model provided in an embodiment of this application.
FIG. 14 is a block diagram of a computer device provided in an embodiment of this application.

### DESCRIPTION OF EMBODIMENTS

To make the objectives, technical solutions, and advantages of this application clearer, implementations of this application will be further described in detail with reference to the accompanying drawings.

A method for training a sustainable learning model in this application involves the following technology:

Artificial intelligence (AI) involves a theory, a method, a technology, and an application system that use a digital computer or a machine controlled by the digital computer to simulate, extend, and expand human intelligence, perceive an environment, acquire knowledge, and use knowledge to obtain an optimal result. In other words, AI is a comprehensive technology in computer science and attempts to understand the essence of intelligence and produce a new intelligent machine that can react in a manner similar to human intelligence. AI is to study the design principles and implementation methods of various intelligent machines, to enable the machines to have the functions of perception, reasoning, and decision-making.

The AI technology is a comprehensive discipline, and relates to a wide range of fields including both hardware-level technologies and software-level technologies. The basic AI technologies generally include technologies such as a sensor, a dedicated AI chip, cloud computing, distributed storage, a big data processing technology, an operating/interaction system, and electromechanical integration. AI software technologies mainly include several major directions such as a computer vision (CV) technology, a speech processing technology, a natural language processing technology, and machine learning/deep learning.

The CV technology is a science that studies how to use a machine to "see", and further, that uses a camera and a computer to replace human eyes to perform machine vision such as recognition and measurement on a target, and further perform graphic processing, so that the computer processes the target into an image more suitable for human eyes to observe, or an image transmitted to an instrument for detection. As a scientific discipline, CV studies related theories and technologies and attempts to establish an AI system that can obtain information from images or multidimensional data. The CV technology generally includes technologies such as image processing, image recognition, image semantic understanding, image retrieval, optical character recognition (OCR), video processing, video semantic understanding, video content/behavior recognition, three-dimensional object reconstruction, a 3 Dimensions (3D) technology, virtual reality, augmented reality and map construction.

Machine learning (ML) is a multi-domain interdiscipline, and involves a plurality of disciplines such as the Probability Theory, the Statistics, the Approximation Theory, the Convex Analysis, and the Algorithm Complexity Theory. ML specializes in studying how a computer simulates or implements a human learning behavior to obtain new knowledge or skills, and reorganize an existing knowledge structure, so as to keep improving its performance. ML is the core of AI, is a basic way to make the computer intelligent, and is applied to various fields of AI. ML and deep learning generally include technologies such as an artificial neural network, a belief network, reinforcement learning, transfer learning, inductive learning, and learning from demonstrations.

With the research and progress of the AI technology, it has been researched and applied in multiple fields, such as common smart homes, smart wearable devices, virtual assistants, smart speakers, intelligent marketing, unmanned driving, autonomous driving, drones, robots, smart healthcare, and intelligent customer service. It is believed that with the development of the technology, the AI technology will be applied in more fields, and plays an increasingly important role.

The solution provided in embodiments of this application involves technologies such as ML and CV in AI, and a lesion region in a pathological image is determined using a trained lesion region determination model.

The technical solutions of this application will be described below with reference to several embodiments.

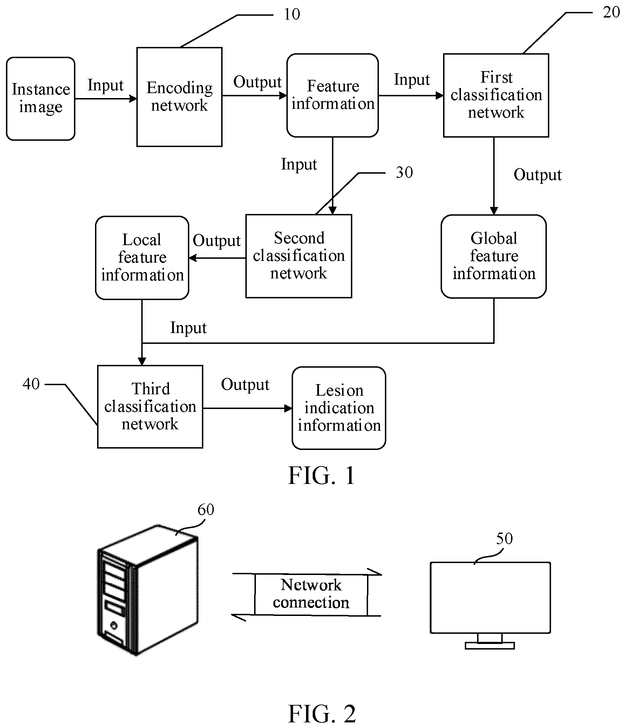

Refer to FIG. 1, which shows a schematic diagram of a model architecture of a lesion region determination model provided in an embodiment of this application. The lesion region determination model may include: an encoding network 10, a first classification network 20, a second classification network 30, and a third classification network 40.

The encoding network 10 is configured to perform feature encoding on an instance image to acquire feature information corresponding to the instance image. Exemplarily, in an embodiment of this application, the above instance image includes at least two first instance images and at least two second instance images. The first instance image is acquired by sampling a pathological image by the first sampling scheme, and the second instance image is acquired by sampling a candidate lesion region by the second sampling scheme. Moreover, in the embodiment of this application, an overlap degree (or a degree of overlap) between the second instance images is greater than that between the first instance images.

The first classification network 20 is configured to determine a first predicted probability corresponding to the first instance image and global feature information of the pathological image according to feature information corresponding to the first instance image, and then determine the lesion region in the pathological image, based on the first predicted probability. The first predicted probability refers to a probability of the presence of the lesion region in the first instance image.

The second classification network 30 is configured to determine local feature information of the pathological image for a candidate lesion region according to feature information corresponding to the second instance image.

The third classification network 40 is configured to determine lesion indication information of the pathological image according to the above global feature information and the above local feature information. The lesion indication information is used for indicating the lesion region in the pathological image. In some alternative or further embodiments, the lesion indication information includes lesion probability distribution information, where the lesion probability distribution information is used for indicating probability distribution of the lesion region in the pathological image

In some embodiments, the above lesion region determination model can be applied to a lesion region determination system. Exemplarily, as shown in FIG. 2, the lesion region determination system includes a terminal device 50 and a server 60.

The terminal device 50 may be an electronic device such as a mobile phone, a tablet, a wearable device, a personal computer (PC), an intelligent voice interaction device, a medical device and a medical assistive robot, which will not be limited in the embodiments of this application. In some embodiments, the terminal device 50 includes a client of an application program. The application program may be any application with a pathological image collection function. Exemplarily, the above application program may be an application program that needs to be downloaded and installed, or a click-to-run application program, including an application program in a web form and an application program in a mini program form, which will not be limited in the embodiments of this application.

The server 60 is configured to provide background services for the terminal device 50. The server 60 may be one server, a server cluster including a plurality of servers, or a cloud computing service center. Optionally, the server 60 may be a background server of the client of the above application program. In an exemplary embodiment, the server 60 provides background services for a plurality of terminal devices 50.

The above terminal device 50 and the above server 60 transmit data via a network. In some embodiments, the server 60 includes a lesion region determination model. The terminal device 50 collects and acquires a pathological image and sends the same to the server 60. Afterwards, the server 60 processes the pathological image, based on the lesion region determination model to determine the lesion region in the pathological image.

The above introduction in FIG. 2 is only illustrative and explanatory. In an exemplary embodiment, functions of the terminal device 50 and the server 60 can be flexibly set and adjusted, which will not be limited in the embodiments of this application. Exemplarily, the above lesion region determination model can also be provided in the terminal device 50. After being acquired by the terminal device 50, a pathological image is processed based on the lesion region determination model to determine a lesion region in the pathological image.

A device for training the above lesion region determination model may be the above server 60, or other computer devices, which will not limited in the embodiments of this application.

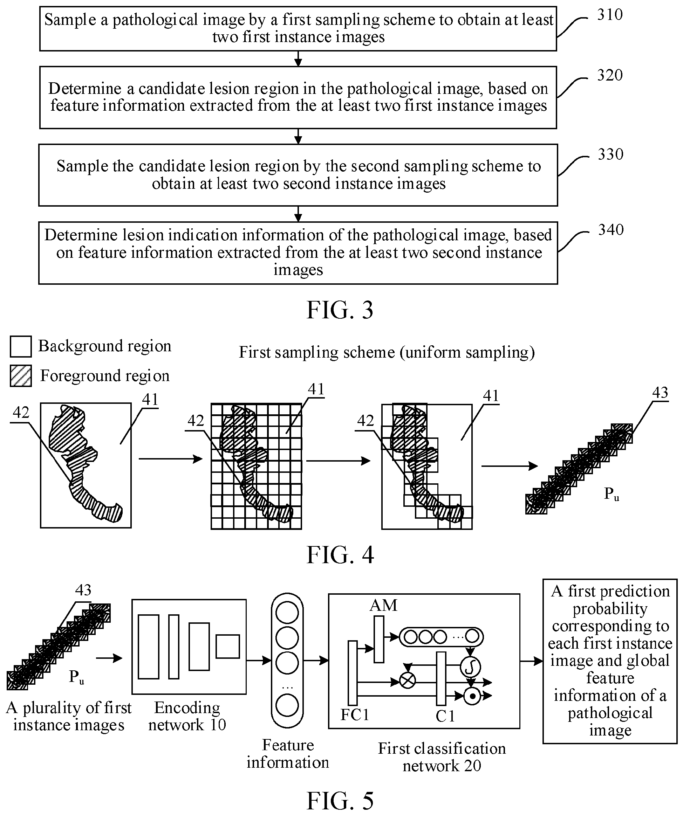

Refer to FIG. 3, which shows a flowchart of a method for determining a lesion region provided in an embodiment of this application. Each step in this method can be executed by the above terminal device 50 and/or the above server 60 (hereinafter collectively referred to as "computer device") in FIG. 2. The method may include the following steps (310 to 340):

step 310: Sample a pathological image by a first sampling scheme to obtain at least two first instance images.

The pathological image is a whole slide image (WSI), which is a type of medical image. In some embodiments, a pathological image is generated by slicing, embedding, staining, scanning, and other processing of tissue or organs in the body of a patient. In an embodiment of this application, after a computer device acquires the above pathological image, the pathological image is sampled by the first sampling scheme to obtain at least two first instance images. In some embodiments, the above pathological image may be a pathological image of teeth, arms, heart, liver, kidneys, lungs, prostate, stomach, and other parts. In some embodiments, the pathological images may be a computed tomography (CT) image, a magnetic resonance imaging (MRI) image, a B-scan ultrasonography image or the like, or other types of pathological images, which will not be specifically limited in the embodiments of this application.

In some embodiments, the pathological image includes a background image and a foreground image. The foreground image refers to an image region corresponding to tissue or organ in the body of a patient, and the background image refers to an image region unrelated to the tissue or organ in the body of the patient. Alternatively, the foreground image refers to an image region of a body part that requires pathological analysis, and the background image refers to the remaining image regions, i.e., image regions other than the image region of the body part that requires the pathological analysis.

Exemplarily, the above first sampling scheme is uniform sampling. The uniform sampling may refer to a sampling scheme of partitioning the plane of a two-dimensional continuous image equidistantly in both horizontal and vertical directions. In an embodiment of this application, the uniform sampling refers to partitioning a pathological image into a plurality of lattices of the same shape and size, where the lattice may be a square, a rectangle, a triangle, a parallelogram or the like, which will not be specifically limited in the embodiment of this application, and these lattices do not overlap with each other. In some embodiments, a first instance image obtained by the uniform sampling is cropped into an instance image of 224 × 224 pixels at 10× magnification.

In some embodiments, step 310 further includes the following sub-steps:
1. Partition the pathological image, to obtain a background image and a foreground image in the pathological image.

In some embodiments, the pathological image is converted to a binary image, and two different colors are used for representing the background image and the foreground image, respectively. For example, there are only black and white colors in the pathological image, where a black image portion represents the background image and a white image portion represents the foreground image. Alternatively, the white image portion represents the background image, and the black image portion represents the foreground image.

2. Uniformly segment the pathological image to obtain at least two first candidate instance images.

In some embodiments, after the background image and the foreground image in the pathological image are determined, uniform sampling is performed on the pathological image, and the pathological image is uniformly segmented into a plurality of candidate instance images. In some embodiments, each candidate instance image obtained by segmentation has the same shape and size.

3. Determine a first candidate instance image including the foreground image from the at least two first candidate instance images as the first instance image.

By partitioning the pathological image into the background image and the foreground image, the image region of the body part that requires the pathological analysis is distinguished from an image region that does not require the pathological analysis, thereby avoiding interference from the image region that does not require the pathological analysis in a subsequent pathological analysis process and helping to reduce subsequent computational complexity.

In some embodiments, a first candidate instance image including the foreground image is determined from the at least two first candidate instance images, and the first candidate instance image including the foreground image is determined as the first instance image.

In some embodiments, for each first candidate instance image obtained after segmentation, if a proportion the foreground image included in the first candidate instance image accounts for in the first candidate instance image is equal to or greater than a first threshold, the first candidate instance image is determined as the first instance image; and if a proportion the foreground image included in the first candidate instance image accounts for in the first candidate instance image is less than the first threshold, it is determined that the first candidate instance image is not the first instance image. The first threshold is equal to or greater than 0%, and the first threshold is less than or equal to 100%. The first threshold may be 0%, 5%, 8%, 10%, 15%, 24%, 30%, 45%, 50%, 62%, 70%, 100% or the like. Of course, the first threshold may also be other numerical values, and a specific numerical value of the first threshold can be set by a related technical person according to the actual situation, which will not be specifically limited in the embodiments of this application.

In some embodiments, there may be only one or more first instance images determined from at least two first candidate instance images, which will not be specifically limited in the embodiments of this application.

In some embodiments, before step 310, the method further includes the following steps: acquiring an initial pathological image; and zooming the initial pathological image to a fixed size to obtain a pathological image. Since the size of the initial pathological image may not be fixed, after the initial pathological image is acquired, the initial pathological image can be firstly zoomed to a set fixed size to obtain a pathological image required in step 310. For example, if the size of the initial pathological image is smaller than the fixed size, the initial pathological image is zoomed-in to the fixed size; and if the size of the initial pathological image is greater than the fixed size, the initial pathological image is zoomed-out to the fixed size. The fixed size can be set by a related technical person according to the actual situation, which will not be specifically limited in the embodiments of this application.

Step 320: Determine a candidate lesion region in the pathological image, based on feature information extracted from the at least two first instance images.

In some embodiments, after a computer device acquires the above first instance image, feature extraction is performed on each first instance image, feature information corresponding to each first instance image is acquired, and based on the feature information corresponding to each first instance image, a candidate lesion region in a pathological image is determined.

The feature information extracted from the at least two first instance images can be used for representing pathological features of the at least two first instance images. The feature information extracted from the at least two first instance images includes first feature information corresponding to each first instance image, and global feature information of the pathological image. The first feature information is used for representing pathological features corresponding to each first instance image, and the global feature information is used for representing global pathological features of the pathological image. For example, feature vectors corresponding to each first instance image (i.e., the feature information of the first instance image) are extracted via a Swin Transformer backbone network.

Global aggregation features of the pathological image and coarse distribution of a lesion region are obtained through the feature information of the first instance image extracted by the first sampling scheme.

The candidate lesion region refers to a region where there is likely a lesion region. The lesion region may refer to a region where there is a tumor, a region where there is carcinogenesis, a region where there is perforation, a region that has ulcerated or has a sign of ulceration, or a region that is clearly abnormal in color. The lesion region may also refer to a region where there are other types of lesions, and can be set by a related technical person, which will not be specifically limited in the embodiments of this application.

Step 330: Sample the candidate lesion region by the second sampling scheme to obtain at least two second instance images.

In some embodiments, after a computer device determines the above candidate lesion region, the candidate lesion region is sampled by the second sampling scheme to obtain at least two second instance images. Optionally, multiple samplings are performed around a given coordinate (such as the center point of the candidate lesion region) to obtain at least two second instance images. An overlap degree (or a second overlap degree) between the second instance images is greater than an overlap degree (or a first overlap degree) between the first instance images, the first overlap degree indicating a first image overlap between the first instance images, and the second overlap degree indicating a second image overlap between the second instance images. For example, there is overlap between the second instance images, and there is no overlap between the first instance images (that is, the first instance images are obtained by the uniform sampling scheme introduced above). For another example, there is an image overlap between the second instance images, as well as between the first instance images, but the overlap degree between the second instance images is greater than that between the first instance images. In some embodiments, the second instance image is an image obtained through dense sampling, and different second instance images may be the same or different in size.

In an embodiment of this application, the overlap degree is used for indicating an overlap extent between images, such as indicating an overlap degree between instance images in a candidate lesion region. In some embodiments, the overlap degree can be worked out by an overlap rate corresponding to each instance image. For example, a mean of overlap rates corresponding to the instance images is determined as an overlap degree of these instance images in a candidate region. Alternatively, a median of overlap rates corresponding to the instance images can be determined as the overlap degree of these instance images in the candidate region.

For each instance image, the overlap rate may refer to a ratio of the total area of overlap regions between this instance image and other instance images to the area of this instance image. In some embodiments, for a second target instance image in at least two second instance images, there is at least one second instance image having an overlap rate with the second target instance image equal to or greater than an overlap threshold. Of course, a specific numerical value of the overlap threshold can also be set by a related technical person according to the actual situation, which will not be specifically limited in the embodiments of this application.

In some embodiments, the overlap degree can also be expressed as a ratio of the sum of the area of all instance images in a candidate lesion region to the area of the candidate lesion region; the overlap degree can also be expressed as a ratio of a difference between the sum of the area of all the instance images in the candidate lesion region and the area of the candidate lesion region over the area of the candidate lesion region; and the overlap degree can also be expressed as a ratio of the total area of overlap regions between every two instance images in the candidate lesion region to the area of the candidate lesion region. If a certain region is both an overlap region between an instance image A and an instance image B and an overlap region between the instance image A and an instance image C, then this region is also an overlap region between the instance image B and instance image C, then when the total area of the overlap regions between every two instance images is calculated, this region is to be calculated three times, that is, the area of this region is multiplied by 3 and then, an obtained product is included in the total area of the overlap regions between every two instance images. Therefore, the worked out total area of the overlap regions between every two instance images may be larger than the area of a candidate region. In some embodiments, the overlap degree is a numerical value equal to or greater than 0, and of course, a value of the overlap degree may also be greater than 1. There may also be other definitions and calculation ways for the overlap degree, and they can be set by a related technical person according to the actual situation, which will not be specifically limited in the embodiments of this application.

In some embodiments, step 330 may further include the following sub-steps:

1. Extract candidate lesion images from the pathological image according to the candidate lesion region.

In some embodiments, after the candidate lesion region in the pathological image is determined, an image of the candidate lesion region can be directly determined as a candidate lesion image. Alternatively, an image of the candidate lesion region plus a surrounding region of the candidate lesion region can be determined as the candidate lesion image. In some embodiments, after the candidate lesion region is determined, the center of the candidate lesion region is taken as the center of the candidate lesion image, a region with the same shape as the pathological image and with the candidate lesion region included is determined, and an image within this region is determined as the candidate lesion image.

2. Zoom the candidate lesion image, based on the size of the pathological image to obtain a target lesion image, where the size of the target lesion image is consistent with that of the pathological image.

**In** some embodiments, if the size of the candidate lesion image is generally smaller than that of the pathological image (i.e., the above fixed size), then the candidate lesion image can be zoomed-in to obtain a target lesion image of which the size is consistent with that of the pathological image.

3. Sample the target lesion image by the second sampling scheme to obtain at least two second instance images.

In some embodiments, the target lesion image is sampled by the above second sampling scheme (such as dense sampling) to obtain at least two second instance images. In some embodiments, the number of the second instance images sampled from the target lesion image is equal to or greater than a second threshold, thereby ensuring a certain sampling number. In some embodiments, a sampling density of the dense sampling is greater than a sampling density of the first sampling scheme, so that more instance images can be obtained from the same target lesion image by using the second sampling scheme than the first sampling scheme.

In the above embodiments, in a case where the shape of the candidate lesion image is the same as that of the pathological image, it is only necessary to zoom-in the candidate lesion image in all directions in the same proportion to obtain the target lesion image with the same size data as the pathological image, without a need to stretch or shorten the size of the candidate lesion image in a certain direction.

Step 340: Determine lesion indication information of the pathological image, based on feature information extracted from the at least two second instance images.

The feature information extracted from the at least two second instance images can be used for representing pathological features of the at least two second instance images. In some embodiments, after a computer device acquires the above second instance images, lesion indication information of a pathological image is determined based on feature information extracted from the above at least two second instance images, where the lesion indication information is used for indicating the lesion region in the pathological image.

In summary, in the technical solution provided in the embodiments of this application, the pathological image is sampled to obtain the instance images, the feature information is extracted from the instance images, and the lesion region in the pathological image is automatically determined based on the feature information of the instance images, thereby reducing the consumption of human resources and saving costs required to determine the lesion region.

In addition, in the embodiments of this application, firstly, the candidate lesion region in the pathological image is determined based on the first sampling scheme, then the second instance image is acquired based on the second sampling scheme with a greater sampling overlap degree than that of the first sampling scheme, and based on the second instance image, the lesion region in the pathological image is determined from the candidate lesion region. That is, global processing is performed on the pathological image with the first instance image, and then local processing is performed on the pathological image with the second instance image. From the global processing to the local processing, the area of a region in the pathological image that needs to be sampled by the second sampling scheme is reduced, thereby reducing the number of the second instance images that need to be collected and feature information extracted, reducing computational resources required to determine the lesion region, and improving the efficiency of determining the lesion region.

Moreover, the candidate lesion region is more likely to include the lesion region than other regions, and has a relatively high signal-to-noise ratio. Thus, by executing the second sampling scheme only for the candidate lesion region, an information loss can be reduced and perception of a lesion region with a relatively small area can be enhanced, thereby determining the lesion region in the pathological image more accurately.

The above way to determine the candidate lesion region will be introduced below.

In some possible implementations, the above step 320 may further include the following steps (1 to 4):

1. Perform feature encoding on each first instance image to obtain first feature information corresponding to each first instance image.

In some embodiments, feature encoding is performed on each first instance image to obtain a feature vector corresponding to each first instance image, where the feature vector corresponding to the first instance image may be a 768-dimensional feature or a feature of other dimensions, which will not be specifically limited in the embodiments of this application.

In some embodiments, the first feature information is extracted from the first instance image via a backbone network, and the process of extracting the first feature information from the first instance image via the backbone network can be described as: ${h}_{k} = Encoder \left({x}_{k}\right)$
where *hₖ* may be a 768-dimensional feature extracted from an instance.

2. Perform feature fusion on the first feature information corresponding to each first instance image to obtain global feature information of the pathological image.

In some embodiments, aggregation (i.e., feature fusion) is performed on the first feature information corresponding to each first instance image via a first classification network to obtain global feature information of the pathological image.

In some embodiments, the first feature information corresponding to each first instance image is processed by an attention mechanism to obtain a weight corresponding to each piece of first feature information; and weighted summation is performed on all pieces of first feature information according to the weight corresponding to each piece of first feature information to obtain global feature information of the pathological image.

In some embodiments, refer to the following formula for the process of obtaining the global feature information of the pathological image: $Logits \left(B\right) = c \left\{g\left({h}_{0},{h}_{1},…,{h}_{k}\right)\right\}$ $z = {\sum }_{i = k}^{K} {a}_{k} {h}_{k}$
where: ${a}_{k} = \frac{exp \left\{{W}^{T}tanh\left(V{h}_{k}^{T}\right)\right\}}{{\sum }_{j = 1}^{K} exp \left\{{W}^{T}tanh\left({Vh}_{j}^{T}\right)\right\}}$
where *Logits*(*B*) is a global feature of the pathological image, c is a first classification network, and *g* is an attention mechanism-based aggregation network; and $W ∈ {R}^{L \times 1}$ , *V* ∈ *R^{L×M}*.

In some embodiments, an attention module corresponding to the attention mechanism allocates a weight to each first instance image (i.e., the first feature information of each first instance image), and performs weighted summation thereon, and an obtained sum serves as a feature vector (i.e., global feature information) representing a package. The global feature information obtained by fusion can be fed into the first classification network to predict a classification label corresponding to each first instance image. The attention module can also be deemed as a binary classification network.

The classification networks (such as the first classification network, the second classification network, and the third classification network) involved in the embodiments of this application can be represented as: $c {\left({h}_{i}\right)}_{n} = {W}_{n} {H}_{i}^{T} , n ∈ \left\{1, 2, 3\right\} , {W}_{n} ∈ {R}^{L \times N}$
where *c*(*hᵢ*)*ₙ* represents an n^{th} classification network.

3. Determine a first predicted probability corresponding to each first instance image according to the global feature information and the first feature information corresponding to each first instance image, where the first predicted probability refers to a probability that the first instance image includes the lesion region.

In some embodiments, after the first feature information corresponding to each first instance image and the global feature information of the pathological image are obtained, a probability that each first instance image includes the lesion region can be determined via the first classification network according to the global feature information and the first feature information corresponding to each first instance image.

In some embodiments, the determining a first predicted probability corresponding to each first instance image according to the global feature information and the first feature information corresponding to each first instance image may include the following sub-steps (3.1 to 3.3):

3.1. Map the global feature information to generate a first probability coefficient, where the first probability coefficient refers to a probability that the pathological image includes the lesion region.

In some embodiments, the global feature information is mapped by Softmax to probability distribution between 0 and 1. For example, a set (also referred to as a package) of all first instance images is defined as B, then *B* = {(*x*₀, *y*₀), (*x*₁, *y*₁), ... , (*xₖ, yₖ*)}, where *xₖ, yₖ* represent a *k*^{th} first instance image and a label thereof, respectively. Then a label *Y*(*B*) of *B* can be defined as: $Y \left(B\right) = \left\{\begin{matrix}0 , if \sum y = 0 \\ 1 , otherwise\end{matrix}\right.$
where *Y*(*B*) = 0 represents the absence of a lesion region in a pathological image, and *Y*(*B*) *=* 1 represents the presence of the lesion region in the pathological image.

3.2. For each first instance image, map the first feature information corresponding to the first instance image to generate a second probability coefficient.

In some embodiments, the first feature information corresponding to each first instance image is mapped by Sigmoid to obtain the second probability coefficient corresponding to each first instance image, where the second probability coefficient refers to an initial probability that the first instance image includes the lesion region.

3.3. Determine a first predicted probability corresponding to the first instance image according to the first probability coefficient and the second probability coefficient.

In some embodiments, the first probability coefficient and the second probability are multiplied to obtain the first predicted probability corresponding to each first instance image. This process can be expressed as: $p \left({h}_{i}\right) = sigmoid \left\{{w}^{T}tanh\left({Vh}_{k}^{T}\right)\right\} softmax \left\{c\left({h}_{i}\right)\right\}$
where *p*(*hᵢ*) represents the first predicted probability of an i^{th} first instance image, *softmax*{*c*(*hᵢ*)} represents the first probability coefficient, and $sigmoid \left\{{w}^{T}tanh\left({Vh}_{k}^{T}\right)\right\}$ represents the second probability coefficient.

In some embodiments, the first predicted probability corresponding to the first instance image can also be expressed as: ${p}_{u} = p \left\{Encoder\left({P}_{u}\right)\right\}$

By using the above method, the first probability coefficient and the second probability coefficient are multiplied to obtain the probability that the first instance image includes the lesion region. Then, a candidate lesion region can be determined according to whether the first predicted probability meets a first condition or not.

4. Determine the candidate lesion region, based on a position of the first instance image corresponding to the first predicted probability that meets a first condition in the pathological image.

In some embodiments, after a first predicted probability corresponding to each first instance image is determined, the first instance image that meets a first condition is selected, and a candidate lesion region is determined based on this.

In some embodiments, the first condition may be that a first predicted probability corresponding to a first instance image is equal to or greater than a third threshold.

In some embodiments, the first condition may also be to sort first instance images with the first predicted probability corresponding to the first instance images not less than x%, such as first instance images with the first predicted probability corresponding to the first instance images not less than 2%, from largest to smallest. That is, the first instance images that meet the first condition refer to the top x% of the first instance images with a maximum predicted probability of the presence of a lesion region. x may be 1, 2, 3 or the like, and a specific numerical value of x can be set by a related technical person according to the actual situation, which will not be specifically limited in the embodiments of this application.

In some embodiments, an image region composed of the first instance images that meet a first condition can be directly determined as a candidate lesion region, or the first instance images that meet the first condition and their surrounding regions can be determined as the candidate lesion region.

By determining a position of the first instance image that meets the first condition in a pathological image as the candidate lesion region, the range of the candidate lesion region is reduced, thereby reducing computational resources required to determine the lesion region and improving the efficiency of determining the lesion region.

The above way to acquire the lesion indication information will be introduced below.

In a possible implementation, the above step 340 may further include the following steps (1 to 3):
1. perform feature encoding on each second instance image to obtain second feature information corresponding to each second instance image;
2. perform feature fusion on the second feature information corresponding to each second instance image to obtain local feature information of the pathological image for a candidate lesion region; and
3. determine lesion probability distribution information of the pathological image, based on the local feature information, and global feature information of the pathological image, where the lesion probability distribution information is used for indicating probability distribution of the lesion region in the pathological image; and where lesion indication information includes the lesion probability distribution information.

By determining the lesion probability distribution information of the pathological image, the probability distribution of the lesion region in the pathological image is obtained, and thereby, the lesion region in the pathological image can be determined based on the lesion probability distribution information, which saves costs of determining the lesion region.

In some embodiments, the performing feature fusion on the second feature information corresponding to each second instance image to obtain local feature information of the pathological image for a candidate lesion region includes: processing the second feature information corresponding to each second instance image by an attention mechanism to obtain a weight corresponding to each piece of second feature information; and performing weighted summation on all pieces of second feature information according to the weight corresponding to each second feature information to obtain local feature information. In some embodiments, an attention module corresponding to the attention mechanism allocates a weight to each second instance image (i.e., the second feature information of each second instance image), and performs weighted summation thereon, and an obtained sum serves as a feature vector (i.e., the local feature information) representing a package. The attention module can also be deemed as a binary classification network.

By performing the weighted summation on all the pieces of second feature information to obtain the local feature information, the local feature information obtained by fusion can be fed into a second classification network to predict a classification label corresponding to each second instance image. In some embodiments, the lesion indication information is obtained by a lesion region determination model, where the lesion region determination model includes an encoding network, a first classification network, a second classification network, and a third classification network; where:
the encoding network is configured to perform feature encoding on the first instance image and the second instance image to obtain the first feature information corresponding to the first instance image and the second feature information corresponding to the second instance image;
the first classification network is configured to determine the first predicted probability corresponding to each first instance image and global feature information of a pathological image according to the first feature information corresponding to each first instance image;
the second classification network is configured to determine the second predicted probability corresponding to each second instance image and local feature information of the pathological image for a candidate lesion region according to the second feature information corresponding to each second instance image; and
the third classification network is configured to determine the lesion probability distribution information of the pathological image according to the global feature information and the local feature information.

Refer to the above embodiment for part of the content of each step in this implementation, which will not be repeated herein.

In some embodiments, sampling is performed by the second sampling scheme to obtain a second instance image, which can be expressed as: ${P}_{r} = Resample \left\{coords\left[argmax\left({p}_{u}, k\right)\right],n\right\}$
where *pᵤ* represents the first predicted probability corresponding to a first instance image, and 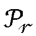 represents the second instance image obtained by sampling by the second sampling scheme.

In some embodiments, after being spliced, the local feature information and the global feature information of the pathological image are inputted into the third classification network to obtain lesion probability distribution information of the pathological image. This process can be expressed as: $Logits \left(B\right) = {c}_{3} \left\{concat\left({z}_{1}, {z}_{2}\right)\right\}$
where *Logits*(*B*) represents the lesion probability distribution information of the pathological image, *z*₁ represents the global feature information, and *z*₂ represents the local feature information.

In another possible implementation, the above step 340 may include the following steps (1 to 4):
1. perform feature encoding on each second instance image to obtain second feature information corresponding to each second instance image;
2. perform feature fusion on the second feature information corresponding to each second instance image to obtain local feature information of the pathological image for a candidate lesion region;
3. determine a second predicted probability corresponding to each second instance image according to the local feature information and the second feature information corresponding to each second instance image, where the second predicted probability refers to a probability that the second instance image includes the lesion region; and
4. determine lesion indication information of the pathological image, based on a position of the second instance image corresponding to the second predicted probability that meets a second condition in the pathological image.

In some embodiments, the second condition may be that the second predicted probability corresponding to the second instance image is equal to or greater than a fourth threshold. The second condition may also be to sort second instance images with the second predicted probability corresponding to the second instance images not less than a second proportion threshold, such as second instance images with the second predicted probability corresponding to the second instance images not less than 15%, from largest to smallest.

In some embodiments, an image region composed of the second instance images that meet a second condition can be directly determined as the lesion region, or the second instance images that meet the second condition and their surrounding regions can be determined as the lesion region.

By determining a position of the second instance image that meets the second condition in a pathological image as lesion indication information, the lesion region in the pathological image is obtained, thereby reducing computational resources required to determine the lesion region and improving the efficiency of determining the lesion region.

In some embodiments, the determining a second predicted probability corresponding to each second instance image according to the local feature information and the second feature information corresponding to each second instance image further includes the following steps (3.1 to 3.3):
3.1. map the local feature information to generate a third probability coefficient, where the third probability coefficient refers to a probability that the candidate lesion region includes the lesion region;
3.2. for each second instance image, map the second feature information corresponding to the second instance image to generate a fourth probability coefficient, where the fourth probability coefficient refers to an initial probability that the second instance image includes the lesion region; and
3.3. determine a second predicted probability corresponding to the second instance image according to the third probability coefficient and the fourth probability coefficient.

In some embodiments, referring to the above embodiments, the local feature information is mapped by Softmax to generate the third probability coefficient; and the second feature information corresponding to the second instance image is mapped by Sigmoid to generate the fourth probability coefficient. In some embodiments, the third probability coefficient and the fourth probability coefficient are multiplied to obtain the second predicted probability corresponding to each second instance image.

Refer to the above embodiment for part of the content of each step in this implementation, which will not be repeated herein.

In this implementation, after the candidate lesion region is determined, the lesion indication information can be predicted directly according to the second instance image, without a need for the global feature information or other information related to the first instance image, thereby simplifying a way to determine the lesion indication information, saving processing resources and time required to determine the lesion indication information, and improving the efficiency of determining the lesion indication information.

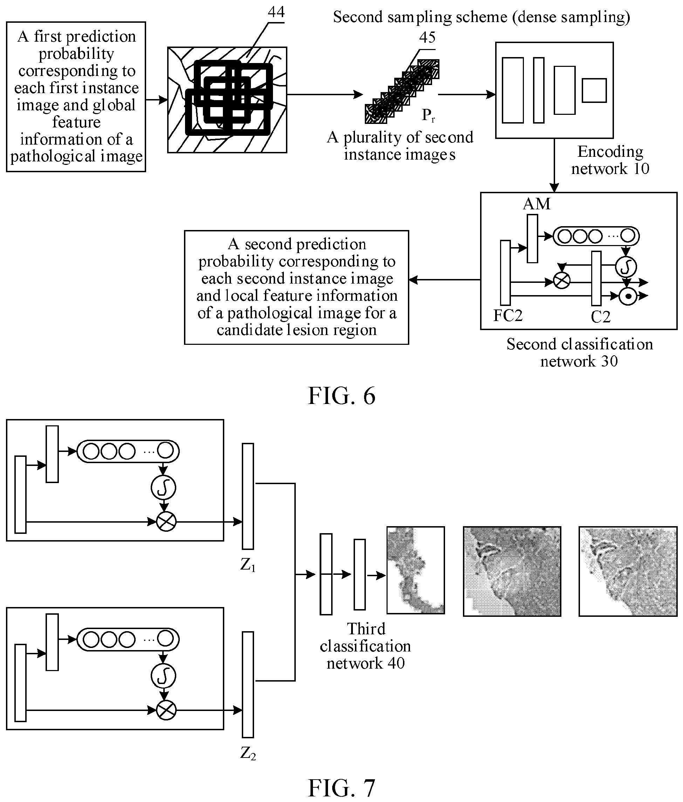

As shown in FIGS. 4 to 7, the method may include the following steps (1 to 4):
1. as shown in FIG. 4, separate a background image 41 from a foreground image 42 in a pathological image, and then uniformly crop the foreground image 42 into a plurality of first instance images 43 by uniform sampling (i.e., the first sampling scheme);
2. as shown in FIG. 5, input the plurality of first instance images 43 into the encoding network 10 and encode the same into 768-dimensional vectors to obtain feature information of the first instance image 43; input the feature information of the first instance image 43 into the first classification network to obtain a first predicted probability corresponding to each first instance image and global feature information of the pathological image, thereby obtaining probability distribution of each local region; and optionally, the encoding network 10 may include convolutional blocks and Swin transformer blocks;
3. as shown in FIG. 6, based on the probability distribution of each local area obtained in the previous step, perform, by a lesion region determination model, dense sampling on a candidate lesion region 44 (i.e., key regions), and encode and infer the candidate lesion region by the second classification network 30 to obtain a second predicted probability corresponding to each second instance image 45 and local feature information of the pathological image for the candidate lesion region 44; and
4. as shown in FIG. 7, concatenate the above results obtained from the second and third steps mentioned above and input a concatenated result into another classification network (i.e. the third classification network) to obtain final lesion indication information.

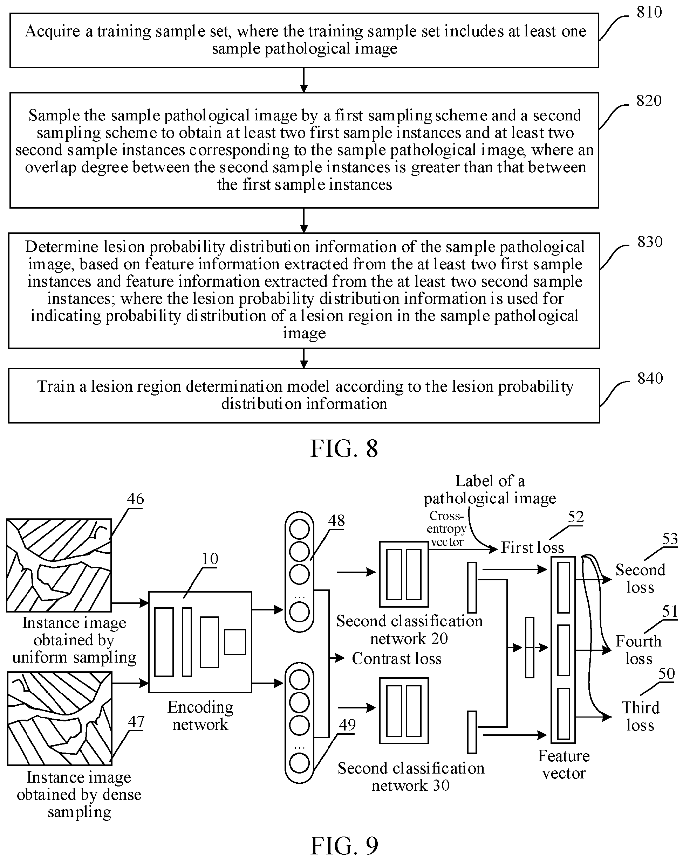

Refer to FIG. 8, which shows a flowchart of a method for training a lesion region determination model provided in another embodiment of this application. Each step in this method can be executed by the above computer device. The method may include the following steps (810 to 840):
step 810: Acquire a training sample set, where the training sample set includes at least one sample pathological image.

In some embodiments, the sample pathological image is a pathological image in which a lesion region has been labeled.

Step 820: Sample the sample pathological image by a first sampling scheme and a second sampling scheme to obtain at least two first sample instances and at least two second sample instances corresponding to the sample pathological image, where a second overlap degree indicating a second image overlap between the second sample instances is greater than a first overlap degree indicating a first image overlap between the first sample instances.

Step 830: Determine lesion probability distribution information of the sample pathological image, based on feature information extracted from the at least two first sample instances and feature information extracted from the at least two second sample instances, the lesion probability distribution information being used for indicating probability distribution of the lesion region in the sample pathological image.

In some embodiments, as shown in FIG. 9, the lesion region determination model includes an encoding network 10, a first classification network 20, a second classification network 30, and a third classification network. Step 830 may include the following steps:
1. perform, by the encoding network 10, feature encoding on each first sample instance 46 and each second sample instance 47 to obtain feature information 48 corresponding to each first sample instance 46 and feature information 49 corresponding to each second sample instance 47;
2. process, by the first classification network 20, the feature information 48 corresponding to each first sample instance 46 to obtain a pseudo label and a first predicted probability corresponding to each first sample instance 46, and global feature information of a sample pathological image; where the first predicted probability refers to a probability that the first sample instance 46 includes the lesion region;
3. process, by the second classification network, the feature information corresponding to each second sample instance 47 to obtain a second predicted probability corresponding to each second sample instance 47, and local feature information of the pathological image; where the second predicted probability refers to a probability that the second sample instance 47 includes the lesion region; and
4. process, by the third classification network, the global feature information and the local feature information to obtain lesion probability distribution information of the sample pathological image. It is noted that reference can be made to the foregoing embodiments of the method for determining a lesion region for specific implementation and description of the above steps 1-4 in step 830.

Step 840: Train the lesion region determination model according to the lesion probability distribution information.

In some embodiments, as shown in FIG. 9, step 840 may include the following steps:
1. generate a first loss 52 according to a pseudo label corresponding to each first sample instance 46, where the first loss 52 is used for measuring the ability of the first classification network 20 to distinguish a positive sample instance from a negative sample instance; the positive sample instance refers to a first sample instance 46 including the lesion region; and the negative sample instance refers to a first sample instance 46 without the lesion region;
2. generate a second loss 53 according to the first predicted probability corresponding to each first sample instance 46, where the second loss 53 is used for measuring the accuracy of a prediction result of the first classification network 20 on whether the first sample instance 46 includes the lesion region or not;
3. generate a third loss 50 according to the second predicted probability corresponding to each second sample instance 47, where the third loss 50 is used for measuring the accuracy of a prediction result of the second classification network 30 on whether the second sample instance 47 includes the lesion region or not;
4. generate a fourth loss 51 according to the lesion probability distribution information, where the fourth loss 51 is used for measuring the accuracy of a prediction result of the third classification network 40 for probability distribution information of the lesion region in the sample pathological image; and
5. train a lesion region determination model according to the first loss 52, the second loss 53, and the third loss 50.

In some embodiments, the lesion region determination model is trained by a self-supervised model training method, such as a Moco V3 method. In theory, two branches of the lesion region determination model (i.e., a branch of processing a first instance image and a branch of processing a second instance image) can both acquire sufficient information to determine whether there is a lesion region in a pathological image or not. Therefore, a label (i.e., a label indicating the presence or absence of a lesion region in a pathological image) of the pathological image may serve as a label for final prediction or an auxiliary signal in a supervised model training process.

In some embodiments, in a training process of a lesion region determination model, a label of a sample pathological image may serve as both a training label and an auxiliary signal in a supervised training process of the lesion region determination model. In some embodiments, from first or second sample instances, k sample instances with a maximum probability of the presence of a lesion region are used as positive sample instances (also referred to as + sample instances), and k sample instances with a minimum probability of the presence of a lesion region are used as negative sample instances (also known as - sample instances), and pseudo labels are generated, and then are constrained with a cross-entropy loss. A loss of a lesion region determination model may be expressed as: ${L}_{i} = - {\sum }_{x} {p}_{i} \left(x\right) log \left\{{q}_{i}\left(x\right)\right\}$
where 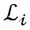 represents a loss of an i^{th} classification network, and 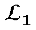 to 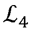 represent losses corresponding to the above first classification network, the above second classification network, the above third classification network, and the above encoding network, respectively.

A total loss of a lesion region determination model may be expressed as: ${L}_{total} = {λ}_{1} {L}_{1} + {λ}_{2} {L}_{2} + {λ}_{3} {L}_{3} + {λ}_{4} {L}_{4}$
where 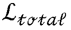 represents a total loss of the lesion region determination model, and λ₁ to λ₄ represent coefficients corresponding to these 4 losses 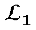 to 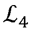 .

In summary, in the technical solution provided in the embodiments of this application, the global processing is performed on the pathological image with the first sample instance, thereby quickly determining the candidate lesion region. Then, the key region (i.e., the candidate lesion region) of the pathological image is processed in a more targeted manner with the second sample instance. The candidate lesion region is more likely to include the lesion region than other regions, and has a relatively high signal-to-noise ratio. Thus, by only processing the candidate lesion region in a second stage, an information loss can be reduced and perception of a lesion region with a relatively small area can be enhanced, so that the trained lesion region determination model can determine the lesion region in the pathological image more accurately, thereby improving the accuracy and precision of the lesion region determination model.

The method for training the lesion region determination model and the method for determining the lesion region provided in the embodiments of this application are a model training process and a model use process corresponding to each other. For details that are not explained in details on one side, refer to the introduction on the other side.

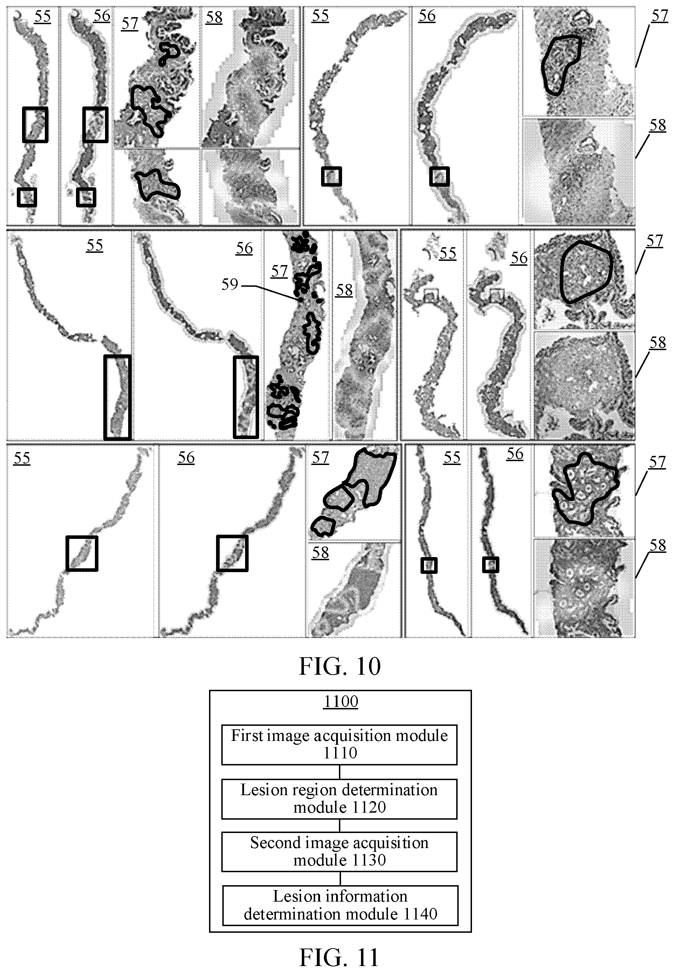

Taking the prostate as an example, through comparative experiments and ablation experiments on a prostate dataset and its difficult sample subset, it can be proven that the technical solution provided in the embodiments of this application has superiority and a good visualization effect. As shown in FIG. 10, for each pathological image, global processing is firstly performed on a pathological image 55 with a first instance image; feature information of the first instance image is extracted by the first sampling scheme, global aggregation features of the pathological image and the coarse distribution information of a lesion region 56 are acquired, and a candidate lesion region 57 is determined; and then by performing local processing on the pathological image (i.e., the candidate lesion region 57) with a second instance image, a probability distribution thermodynamic chart 58 of the candidate lesion region 57 is obtained. From FIG. 10, it can be seen that even for a relatively small lesion region (such as a lesion region 59), it can also be accurately identified by the technical solution provided in the embodiments of this application. It can be seen that the embodiments of this application have a relatively good recall rate for relatively small lesion regions (such as tumorlets), that is, the solution provided in the embodiments of this application may also have a relatively good recall rate for difficult sample images.

As shown in Table 1 below, by employing test sets of the technical solution provided in the embodiments of this application in comparative experiments, indicator data in all aspects were significantly superior to those of comparative cases. It can be seen that compared to the comparative case, the technical solution provided in the embodiments of this application has higher accuracy and precision in terms of determining the lesion region in the pathological image.

**Table 1. Results of Comparative Experiments and Ablation Experiments**

| Data from full test sets and comparative case sets for comparative experiments and the ablation experiments | | | | | |
|---|---|---|---|---|---|
| Data set | Method | Comprehensive evaluation indexes | AUC (accuracy) | SEN | SPE |
| Test set | CLAM[10] | 0.9205±0.0029 | 0.9500±0.0014 | 0.8921±0.0084 | 0.8789±0.0124 |
| | ABMIL[16] | 0.9188±0.0070 | 0.9535±0.0012 | 0.8779±0.0180 | 0.9136±0.0211 |
| | DSMIL[11] | 0.9160±0.0032 | 0.9387±0.0050 | 0.8998±0.0143 | 0.8294±0.0484 |
| | B1 only | 0.9575±0.0023 | 0.9824±0.0007 | 0.9381±0.0059 | 0.9438±0.0072 |
| | B2 only | 0.9560±0.0038 | 0.9832±0.0012 | 0.9303±0.0089 | 0.9585±0.0093 |
| | Global IS | 0.9570±0.0021 | 0.9819±0.0008 | 0.9477±0.0055 | 0.9136±0.0140 |
| | IS-MIL | 0.9654±0.0026 | 0.9869±0.0008 | 0.9506±0.0036 | 0.9512±0.0058 |
| Comparative cases | CLAM[10] | 0.4383±0.0270 | 0.5631±0.0089 | 0.3661±0.0344 | 0.7358±0.0200 |
| | ABMIL[16] | 0.4072±0.0512 | 0.5605±0.0168 | 0.3267±0.0566 | 0.7654±0.0302 |
| | DSMIL[11] | 0.4635±0.0418 | 0.5460±0.0171 | 0.4253±0.0725 | 0.6518±0.0778 |
| | B1 only | 0.4750±0.0635 | 0.6455±0.0430 | 0.3971±0.0763 | 0.7703±0.0228 |
| | B2 only | 0.5303±0.0497 | 0.7313±0.0139 | 0.4224±0.0590 | 0.8467±0.0318 |
| | Global IS | 0.6285±0.0180 | 0.7424±0.0054 | 0.6000±0.0418 | 0.7308±0.0457 |
| | IS-MIL | 0.6339±0.0393 | 0.7532±0.0314 | 0.5690±0.0633 | 0.8049±0.0343 |

The following describes apparatus embodiments of this application, which can be used for executing the method embodiments of this application. For details not disclosed in the apparatus embodiments of this application, refer to the method embodiments of this application.

Refer to FIG. 11, which shows a block diagram of an apparatus for determining a lesion region provided in an embodiment of this application. The apparatus has a function of implementing the above method for determining the lesion region, where the function may be implemented by hardware or may be implemented by hardware executing corresponding software. The apparatus may be a computer device or can be provided therein. The apparatus 1100 may include: a first image acquisition module 1110, a lesion region determination module 1120, a second image acquisition module 1130 and a lesion information determination module 1140.

The first image acquisition module 1110 is configured to sample a pathological image by a first sampling scheme to obtain at least two first instance images, the first instance images having a first image overlap indicated by a first overlap degree.

The lesion region determination module 1120 is configured to determine a candidate lesion region in the pathological image, based on feature information extracted from the at least two first instance images.

The second image acquisition module 1130 is configured to sample the candidate lesion region by the second sampling scheme to obtain at least two second instance images, the second instance images having a second image overlap indicated by a second overlap degree higher than the first overlap degree.

The lesion information determination module 1140 is configured to determine lesion indication information of the pathological image, based on feature information extracted from the at least two second instance images, where the lesion indication information is used for indicating the lesion region in the pathological image.

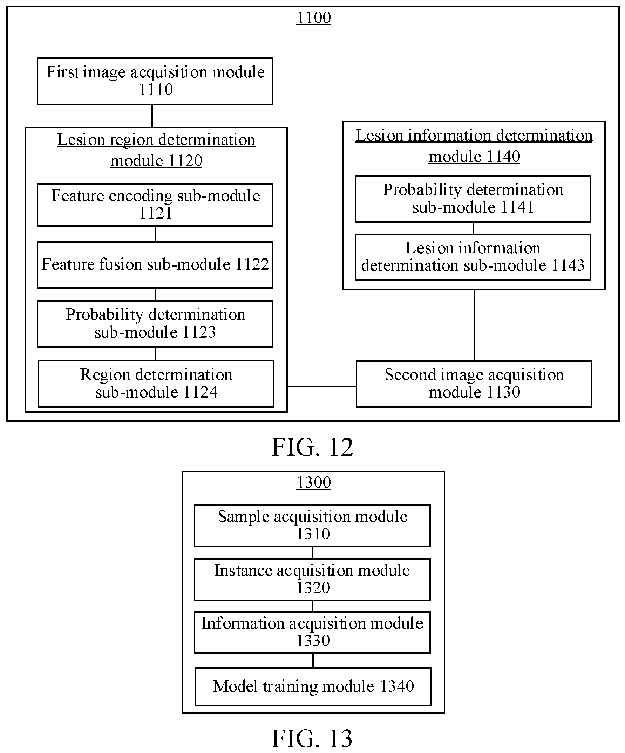

In some embodiments, as shown in FIG. 12, the lesion region determination module 1120 includes: a feature encoding sub-module 1121, a feature fusion sub-module 1122, a probability determination sub-module 1123, and a region determination sub-module 1124.

The feature encoding sub-module 1121 is configured to perform feature encoding on each first instance image to obtain first feature information corresponding to each first instance image.

The feature fusion sub-module 1122 is configured to perform feature fusion on the first feature information corresponding to each first instance image to obtain global feature information of the pathological image.

The probability determination sub-module 1123 is configured to determine a first predicted probability corresponding to each first instance image according to the global feature information and the first feature information corresponding to each first instance image, where the first predicted probability refers to a probability that the first instance image includes the lesion region.

The region determination sub-module 1124 is configured to determine a candidate lesion region, based on a position of the first instance image corresponding to the first predicted probability that meets a first condition in the pathological image.

In some embodiments, as shown in FIG. 12, the feature fusion sub-module 1122 is configured to:
process the first feature information corresponding to each first instance image by an attention mechanism to obtain a weight corresponding to each piece of first feature information; and
perform weighted summation on all the pieces of first feature information according to the weight corresponding to each piece of first feature information to obtain global feature information of the pathological image.

In some embodiments, as shown in FIG. 12, the probability determination sub-module 1123 is configured to:
map the global feature information to generate a first probability coefficient, where the first probability coefficient refers to a probability that the pathological image includes the lesion region;
for each first instance image, map first feature information corresponding to the first instance image to generate a second probability coefficient, where the second probability coefficient refers to an initial probability that the first instance image includes the lesion region; and
determine a first predicted probability corresponding to the first instance image according to the first probability coefficient and the second probability coefficient.

In some embodiments, as shown in FIG. 12, the lesion information determination module 1140 includes: a probability determination sub-module 1141.

The feature encoding sub-module 1121 is further configured to perform feature encoding on each second instance image to obtain second feature information corresponding to each second instance image.

The feature fusion sub-module 1122 is further configured to perform feature fusion on the second feature information corresponding to each second instance image to obtain local feature information of the pathological image for the candidate lesion region.

The probability determination sub-module 1141 is configured to determine lesion probability distribution information of the pathological image, based on the local feature information, and global feature information of the pathological image, where the lesion probability distribution information is used for indicating probability distribution of the lesion region in the pathological image; and where the lesion indication information includes the lesion probability distribution information.

In some embodiments, as shown in FIG. 12, the feature fusion sub-module 1122 is configured to:
process the second feature information corresponding to each second instance image by an attention mechanism to obtain a weight corresponding to each piece of second feature information; and
perform weighted summation on all the pieces of second feature information according to the weight corresponding to each second feature information to obtain the local feature information.

In some embodiments, as shown in FIG. 12, the lesion information determination module 1140 includes: a lesion information determination sub-module 1142.

The feature encoding sub-module 1121 is further configured to perform feature encoding on each second instance image to obtain second feature information corresponding to each second instance image.

The feature fusion sub-module 1122 is further configured to perform feature fusion on the second feature information corresponding to each second instance image to obtain local feature information of the pathological image for the candidate lesion region.

The probability determination sub-module 1141 is configured to determine a second predicted probability corresponding to each second instance image according to the local feature information and the second feature information corresponding to each second instance image, where the second predicted probability refers to a probability that the second instance image includes the lesion region.

The lesion information determination sub-module 1142 is configured to determine lesion indication information of the pathological image, based on a position of the second instance image corresponding to the second predicted probability that meets a second condition in the pathological image.

In some embodiments, as shown in FIG. 12, the probability determination sub-module 1142 is configured to:
map the local feature information to generate a third probability coefficient, where the third probability coefficient refers to a probability that the candidate lesion region includes the lesion region;
for each second instance image, map the second feature information corresponding to the second instance image to generate a fourth probability coefficient, where the fourth probability coefficient refers to an initial probability that the second instance image includes the lesion region; and
determine a second predicted probability corresponding to the second instance image according to the third probability coefficient and the fourth probability coefficient.

In some embodiments, the first image acquisition module 1110 is configured to:
partition a background of the pathological image, and determine a background image and a foreground image in the pathological image;
segment the pathological image by the first sampling scheme to obtain at least two first candidate instance images; and
determine a first candidate instance image including the foreground image from the at least two first candidate instance images as the first instance image.

In some embodiments, the second image acquisition module 1130 is configured to:
extract candidate lesion images from the pathological image according to the candidate lesion region;
zoom the candidate lesion image, based on the size of the pathological image to obtain a target lesion image, where the size of the target lesion image is consistent with that of the pathological image; and
sample the target lesion image by the second sampling scheme to obtain the at least two second instance images.

In some embodiments, the lesion indication information is obtained by a lesion region determination model, where the lesion region determination model includes an encoding network, a first classification network, a second classification network, and a third classification network; where
the encoding network is configured to perform feature encoding on the first instance image and the second instance image to obtain the first feature information corresponding to the first instance image and the second feature information corresponding to the second instance image;
the first classification network is configured to determine the first predicted probability corresponding to each first instance image and the global feature information of the pathological image according to the first feature information corresponding to each first instance image;
the second classification network is configured to determine the second predicted probability corresponding to each second instance image and the local feature information of the pathological image for the candidate lesion region according to the second feature information corresponding to each second instance image; and
the third classification network is configured to determine the lesion probability distribution information of the pathological image according to the global feature information and the local feature information.

In summary, in the technical solution provided in the embodiments of this application, the pathological image is sampled to obtain the instance images, the feature information is extracted from the instance images, and the lesion region in the pathological image is automatically determined based on the feature information of the instance images, thereby reducing the consumption of human resources and saving costs required to determine the lesion region.

In addition, in the embodiments of this application, firstly, the candidate lesion region in the pathological image is determined based on the first sampling scheme, then the second instance image is acquired based on the second sampling scheme with a greater sampling overlap degree than that of the first sampling scheme, and based on the second instance image, the lesion region in the pathological image is determined from the candidate lesion region. Thus the area of a region in the pathological image that needs to be sampled by the second sampling scheme is reduced, thereby reducing the number of the second instance images that need to be collected and feature information extracted, reducing computational resources required to determine the lesion region, and improving the efficiency of determining the lesion region.

Moreover, the candidate lesion region is more likely to include the lesion region than other regions, and has a relatively high signal-to-noise ratio. Thus, by executing the second sampling scheme only for the candidate lesion region, an information loss can be reduced and perception of a lesion region with a relatively small area can be enhanced, thereby determining the lesion region in the pathological image more accurately.

Refer to FIG. 13, which shows a block diagram of an apparatus for training a lesion region determination model provided in an embodiment of this application. The apparatus has a function of implementing the above method for training a lesion region determination model, where the function may be implemented by hardware or may be implemented by hardware executing corresponding software. The apparatus may be a computer device or can be provided therein. The apparatus 1300 may include: a sample acquisition module 1310, an instance acquisition module 1320, an information acquisition module 1330, and a model training module 1340.

The sample acquisition module 1310 is configured to acquire a training sample set, where the training sample set includes at least one sample pathological image.

The instance acquisition module 1320 is configured to sample the sample pathological image by a first sampling scheme and a second sampling scheme to obtain at least two first sample instances and at least two second sample instances corresponding to the sample pathological image, a second overlap degree indicating a second image overlap between the second sample instances being greater than a first overlap degree indicating a first image overlap between the first sample instances.

The information acquisition module 1330 is configured to determine lesion probability distribution information of the sample pathological image, based on feature information extracted from the at least two first sample instances and feature information extracted from the at least two second sample instances, the lesion probability distribution information being used for indicating probability distribution of the lesion region in the sample pathological image.

The model training module 1340 is configured to train the lesion region determination model according to the lesion probability distribution information.

In some embodiments, the lesion region determination model includes an encoding network, a first classification network, a second classification network, and a third classification network. The information acquisition module 1330 is configured to:
perform, by the encoding network, feature encoding on each first sample instance and each second sample instance to obtain the feature information corresponding to each first sample instance and the feature information corresponding to each second sample instance;
process, by the first classification network, the feature information corresponding to each first sample instance to obtain a pseudo label and a first predicted probability corresponding to each first sample instance, and global feature information of the sample pathological image; where the first predicted probability refers to a probability that the first sample instance includes the lesion region;
process, by the second classification network, the feature information corresponding to each second sample instance to obtain a second predicted probability corresponding to each second sample instance, and local feature information of the pathological image; where the second predicted probability refers to a probability that the second sample instance includes the lesion region; and
process, by the third classification network, the global feature information and the local feature information to obtain the lesion probability distribution information of the sample pathological image.

In some embodiments, the model training module 1340 is configured to:
generate a first loss according to a pseudo label corresponding to each first sample instance, where the first loss is used for measuring the ability of the first classification network to distinguish a positive sample instance from a negative sample instance; the positive sample instance refers to a first sample instance including the lesion region; and the negative sample instance refers to a first sample instance without the lesion region;
generate a second loss according to the first predicted probability corresponding to each first sample instance, where the second loss is used for measuring the accuracy of a prediction result of the first classification network on whether the first sample instance includes the lesion region or not;
generate a third loss according to the second predicted probability corresponding to each second sample instance, where the third loss is used for measuring the accuracy of a prediction result of the second classification network on whether the second sample instance includes the lesion region or not;
generate a fourth loss according to the lesion probability distribution information, where the fourth loss is used for measuring the accuracy of a prediction result of the third classification network for probability distribution information of the lesion region in the sample pathological image; and
train the lesion region determination model according to the first loss, the second loss, the third loss and the fourth loss.

In summary, in the technical solution provided in the embodiments of this application, the global processing is performed on the pathological image with the first sample instance, thereby quickly determining the candidate lesion region. Then, the key region (i.e., the candidate lesion region) of the pathological image is processed in a more targeted manner with the second sample instance. The candidate lesion region is more likely to include the lesion region than other regions, and has a relatively high signal-to-noise ratio. Thus, by only processing the candidate lesion region in a second stage, an information loss can be reduced and perception of a lesion region with a relatively small area can be enhanced, so that the trained lesion region determination model can determine the lesion region in the pathological image more accurately, thereby improving the accuracy and precision of the lesion region determination model.

When the apparatus provided in the above embodiment implements its functions, it is only illustrated with the division of the above functional modules as an example. In the practical application, the above functions may be allocated to and completed by different function modules according to requirements. That is, an internal structure of the device is divided into different function modules to complete all or some of the functions described above. In addition, the apparatus provided in the above embodiments and the method embodiments fall within the same conception. For details of a specific implementation process, refer to the method embodiments, which will not repeated herein.

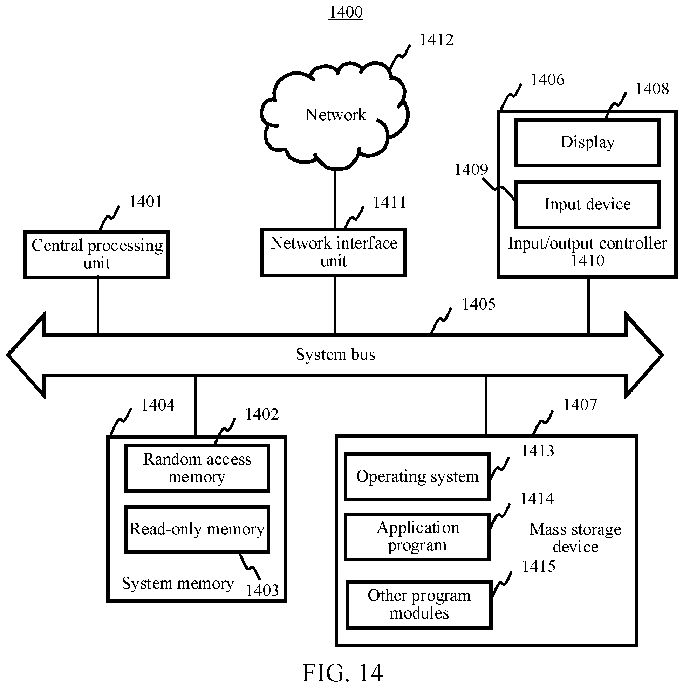

Refer to FIG. 14, which shows a structural block diagram of a computer device provided in an embodiment of this application. The computer device can be configured to implement the function of the above method for determining the lesion region, or to implement the function of the above method for training the lesion region determination model. Specifically:

the computer device 1400 includes a central processing unit (CPU) 1401, a system memory 1404 including a random access memory (RAM) 1402 and a read only memory (ROM) 1403, and a system bus 1405 connecting the system memory 1404 and the CPU 1401. The computer device 1400 further includes a basic input/output (I/O) system 1406 assisting in transmitting information between components in a computer, and a mass storage device 1407 configured to store an operating system 1413, an application program 1414, and other program modules 1415.

The basic I/O system 1406 includes a display 1408 configured to display information and an input device 1409 such as a mouse or a keyboard that is configured to input information by a user. The display 1408 and the input device 1409 are both connected to the CPU 1401 via an input/output controller 1410 connected to the system bus 1405. The basic I/O system 1406 may further include the input and output controller 1410 to receive and process inputs from a plurality of other devices such as a keyboard, a mouse, and an electronic stylus. Similarly, the input/output controller 1410 further provides an output to a display screen, a printer, or other types of output devices.

The mass storage device 1407 is connected to the CPU 1401 via a mass storage controller (not shown) connected to the system bus 1405. The mass storage device 1407 and a computer-readable medium associated with the large-capacity storage device provide non-volatile storage to the computer device 1400. That is, the mass storage device 1407 may include a computer-readable medium (not shown) such as a hard disk or a compact disc read-only memory (CD-ROM) drive.

Without loss of generality, the computer-readable medium may include a computer storage medium and a communication medium. The computer storage medium includes volatile and non-volatile media, and removable and non-removable media implemented with any method or technology used for storing information such as computer-readable instructions, data structures, program modules, or other data. The computer storage medium includes an RAM, an ROM, an erasable programmable read only memory (EPROM), an electrically erasable programmable read only memory (EEPROM), a flash memory or other solid-state storage devices, a CD-ROM, a digital video disc (DVD) or other optical memories, a tape cartridge, a magnetic cassette, a magnetic disk memory, or other magnetic storage devices. Of course, a person skilled in art can know that the computer storage medium is not limited to the above several types. The above system memory 1404 and the above mass storage device 1407 may be collectively referred to as a memory.

According to the various embodiments of this application, the computer device 1400 may further be connected, through a network such as the Internet, to a remote computer on the network and run. That is, the computer device 1400 may be connected to a network 1412 by using a network interface unit 1411 connected to the system bus 1405, or may be connected to another type of network or a remote computer system (not shown) by using a network interface unit 1411.

The memory further includes a computer program, where the computer program is stored in the memory, and is configured to be executed by one or more processors to implement the above method for determining a lesion region or the above method for training a lesion region determination model.

In an exemplary embodiment, a computer-readable storage medium is further provided, where the storage medium stores a computer program therein, and the computer program, when executed by a processor, implements the above method for determining the lesion region or the above method for training the lesion region determination model.

Optionally, the computer-readable storage medium may include: a read-only memory (ROM), a random access memory (RAM), a solid state drive (SSD), and an optical disc. The RAM may include a resistance random access memory (ReRAM) and a dynamic random access memory (DRAM).

In an exemplary embodiment, a computer program product is further provided, where the computer program product includes a computer program, and the computer program is stored in a computer-readable storage medium. A processor of the computer device reads the computer program from the computer-readable storage medium, and the processor executes the computer program such that the computer device executes the above method for determining the lesion region or executes the above method for training the lesion region determination model.

"A plurality of" mentioned herein refers to two or more. "And/or" describes an association relationship between associated objects and represents that there may be three relationships. For example, A and/or B may represent the following three cases: only A exists, both A and B exist, and only B exists. The character "/" generally indicates an "or" relationship between the associated objects. In addition, the step numbers described herein merely exemplarily show a possible execution sequence of the steps. In some other embodiments, the above steps may not be performed according to the number sequence. For example, two steps with different numbers may be performed simultaneously, or two steps with different numbers may be performed according to a sequence contrary to the sequence shown in the figure. This is not limited in the embodiments of this application.

The above descriptions are merely exemplary embodiments of this application, but are not intended to limit this application.

## Claims

1. A method for determining a lesion region, the method being executable by a computer device, and comprising:
sampling (310) a pathological image by a first sampling scheme to obtain at least two first instance images, the first instance images having a first image overlap indicated by a first overlap degree;
determining (320) a candidate lesion region in the pathological image, based on feature information extracted from the at least two first instance images;
sampling (330) the candidate lesion region by a second sampling scheme to obtain at least two second instance images, the second instance images having a second image overlap indicated by a second overlap degree higher than the first overlap degree; and
determining (340) lesion indication information of the pathological image, based on feature information extracted from the at least two second instance images, the lesion indication information indicating the lesion region in the pathological image, wherein the determining (320) a candidate lesion region in the pathological image, based on feature information extracted from the at least two first instance images comprises:
performing feature encoding on each first instance image to obtain first feature information of each first instance image;
performing feature fusion on the first feature information of each first instance image to obtain global feature information of the pathological image;
determining a first predicted probability corresponding to each first instance image according to the global feature information and the first feature information of each first instance image, wherein the first predicted probability refers to a probability that the first instance image comprises the lesion region; and
determining the candidate lesion region, based on a position of the first instance image corresponding to the first predicted probability that meets a first condition in the pathological image;
wherein the determining (340) lesion indication information of the pathological image, based on feature information extracted from the at least two second instance images comprises:
performing feature encoding on each second instance image to obtain second feature information of each second instance image;
performing feature fusion on the second feature information of each second instance image to obtain local feature information of the pathological image for the candidate lesion region;
determining a second predicted probability corresponding to each second instance image according to the local feature information and the second feature information of each second instance image, wherein the second predicted probability refers to a probability that the second instance image comprises the lesion region; and
determining lesion indication information of the pathological image, based on a position of the second instance image corresponding to the second predicted probability that meets a second condition in the pathological image.

2. The method according to claim 1, wherein the performing feature fusion on the first feature information of each first instance image to obtain global feature information of the pathological image comprises:
processing the first feature information of each first instance image by an attention mechanism to obtain a weight of each piece of first feature information; and
performing weighted summation on all the pieces of first feature information according to the weight of each piece of first feature information to obtain global feature information of the pathological image.

3. The method according to claim 1 or 2, wherein the determining a first predicted probability corresponding to each first instance image according to the global feature information and the first feature information of each first instance image comprises:
mapping the global feature information to generate a first probability coefficient, wherein the first probability coefficient refers to a probability that the pathological image comprises the lesion region;
for each first instance image, mapping first feature information of the first instance image to generate a second probability coefficient, wherein the second probability coefficient refers to an initial probability that the first instance image comprises the lesion region; and
determining a first predicted probability corresponding to the first instance image according to the first probability coefficient and the second probability coefficient.

4. The method according to any one of claims 1 to 3, wherein the determining (340) lesion indication information of the pathological image, based on feature information extracted from the at least two second instance images comprises:
performing feature encoding on each second instance image to obtain second feature information of each second instance image;
performing feature fusion on the second feature information of each second instance image to obtain local feature information of the pathological image for the candidate lesion region; and
determining lesion probability distribution information of the pathological image, based on the local feature information, and global feature information of the pathological image, the lesion probability distribution information indicating probability distribution of the lesion region in the pathological image; wherein the lesion indication information comprises the lesion probability distribution information.

5. The method according to claim 4, wherein the performing feature fusion on the second feature information of each second instance image to obtain local feature information of the pathological image for the candidate lesion region comprises:
processing the second feature information of each second instance image by an attention mechanism to obtain a weight of each piece of second feature information; and
performing weighted summation on all the pieces of second feature information according to the weight of each second feature information to obtain the local feature information.

6. The method according to claim 1, wherein the determining a second predicted probability corresponding to each second instance image according to the local feature information and the second feature information of each second instance image comprises:
mapping the local feature information to generate a third probability coefficient, wherein the third probability coefficient refers to a probability that the candidate lesion region comprises the lesion region;
for each second instance image, mapping the second feature information of the second instance image to generate a fourth probability coefficient, wherein the fourth probability coefficient refers to an initial probability that the second instance image comprises the lesion region; and
determining a second predicted probability corresponding to the second instance image according to the third probability coefficient and the fourth probability coefficient.

7. The method according to any one of claims 1 to 6, wherein the sampling (310) a pathological image by a first sampling scheme to obtain at least two first instance images comprises:
partitioning the pathological image, to obtain a background image and a foreground image in the pathological image;
segmenting the pathological image by the first sampling scheme to obtain at least two first candidate instance images; and
determining a first candidate instance image comprising the foreground image from the at least two first candidate instance images as the first instance image.

8. The method according to any one of claims 1 to 7, wherein the sampling (330) the candidate lesion region by a second sampling scheme to obtain at least two second instance images comprises:
extracting candidate lesion images from the pathological image according to the candidate lesion region;
zooming the candidate lesion image, based on the size of the pathological image to obtain a target lesion image, wherein a size of the target lesion image is consistent with a size of the pathological image; and
sampling the target lesion image by the second sampling scheme to obtain the at least two second instance images.

9. The method according to any one of claims 1 to 8, wherein the lesion indication information is obtained by a lesion region determination model, the lesion region determination model comprising an encoding network, a first classification network, a second classification network, and a third classification network; wherein
the encoding network is configured to perform feature encoding on the first instance image and the second instance image to obtain the first feature information of the first instance image and the second feature information of the second instance image;
the first classification network is configured to determine the first predicted probability corresponding to each first instance image and the global feature information of the pathological image according to the first feature information of each first instance image;
the second classification network is configured to determine the second predicted probability of each second instance image and the local feature information of the pathological image for the candidate lesion region according to the second feature information of each second instance image; and
the third classification network is configured to determine the lesion probability distribution information of the pathological image according to the global feature information and the local feature information.

10. A method for training a lesion region determination model, the method being executable by a computer device, and comprising:
acquiring (810) a training sample set, the training sample set comprising at least one sample pathological image;
sampling (820) the sample pathological image by a first sampling scheme and a second sampling scheme to obtain at least two first sample instances and at least two second sample instances of the sample pathological image, a second overlap degree indicating a second image overlap between the second sample instances being greater than a first overlap degree indicating a first image overlap between the first sample instances;
determining (830) lesion probability distribution information of the sample pathological image, based on feature information extracted from the at least two first sample instances and feature information extracted from the at least two second sample instances, the lesion probability distribution information indicating probability distribution of the lesion region in the sample pathological image; and
training (840) the lesion region determination model according to the lesion probability distribution information, wherein the lesion region determination model comprises an encoding network, a first classification network, a second classification network, and a third classification network; and
the determining (830) lesion probability distribution information of the sample pathological image, based on feature information extracted from the at least two first sample instances and feature information extracted from the at least two second sample instances comprises:
performing, by the encoding network, feature encoding on each first sample instance and each second sample instance to obtain the feature information of each first sample instance and the feature information of each second sample instance;
processing, by the first classification network, the feature information of each first sample instance to obtain a pseudo label and a first predicted probability corresponding to each first sample instance, and global feature information of the sample pathological image; wherein the first predicted probability refers to a probability that the first sample instance comprises the lesion region;
processing, by the second classification network, the feature information of each second sample instance to obtain a second predicted probability corresponding to each second sample instance, and local feature information of the pathological image; wherein the second predicted probability refers to a probability that the second sample instance comprises the lesion region; and
processing, by the third classification network, the global feature information and the local feature information to obtain the lesion probability distribution information of the sample pathological image.

11. A computer device, comprising a processor and a memory, the memory storing a computer program therein, and the computer program being loaded and executed by the processor to implement the method for determining a lesion region according to any one of claims 1 to 9, or the method for training a lesion region determination model according to claim 10.

12. A computer-readable storage medium storing a computer program therein, the computer program being loaded and executed by a processor to implement the method for determining a lesion region according to any one of claims 1 to 9, or the method for training a lesion region determination model according to claim 10.

## Patentansprüche

1. Verfahren zur Bestimmung eines Läsionsbereichs, wobei das Verfahren durch eine Computervorrichtung ausführbar ist und umfasst:
Abtasten (310) eines pathologischen Bildes durch ein erstes Abtastschema, um mindestens zwei erste Instanzbilder zu erhalten, wobei die ersten Instanzbilder eine erste Bildüberlappung aufweisen, die durch einen ersten Überlappungsgrad angegeben wird;
Bestimmen (320) eines Läsionskandidatenbereichs in dem pathologischen Bild auf der Grundlage von Merkmalsinformationen, die aus den mindestens zwei ersten Instanzbildern extrahiert werden;
Abtasten (330) des Läsionskandidatenbereichs durch ein zweites Abtastschema, um mindestens zwei zweite Instanzbilder zu erhalten, wobei die zweiten Instanzbilder eine zweite Bildüberlappung aufweisen, die durch einen zweiten Überlappungsgrad angegeben wird, der höher ist als der erste Überlappungsgrad; und
Bestimmen (340) von Läsionsindikationsinformationen des pathologischen Bildes auf der Grundlage von Merkmalsinformationen, die aus den mindestens zwei zweiten Instanzbildern extrahiert werden, wobei die Läsionsindikationsinformationen den Läsionsbereich in dem pathologischen Bild angeben, wobei das Bestimmen (320) eines Läsionskandidatenbereichs in dem pathologischen Bild auf der Grundlage von Merkmalsinformationen, die aus den mindestens zwei ersten Instanzbildern extrahiert werden, umfasst:
Durchführen einer Merkmalskodierung an jedem ersten Instanzbild, um erste Merkmalsinformationen jedes ersten Instanzbildes zu erhalten;
Durchführen einer Merkmalsfusion an den ersten Merkmalsinformationen jedes ersten Instanzbildes, um globale Merkmalsinformationen des pathologischen Bildes zu erhalten;
Bestimmen einer ersten vorhergesagten Wahrscheinlichkeit entsprechend jedem ersten Instanzbild gemäß den globalen Merkmalsinformationen und den ersten Merkmalsinformationen jedes ersten Instanzbildes, wobei sich die erste vorhergesagte Wahrscheinlichkeit auf eine Wahrscheinlichkeit bezieht, dass das erste Instanzbild den Läsionsbereich umfasst; und
Bestimmen des Läsionskandidatenbereichs auf der Grundlage einer Position des ersten Instanzbildes, das der ersten vorhergesagten Wahrscheinlichkeit entspricht, die eine erste Bedingung in dem pathologischen Bild erfüllt;
wobei das Bestimmen (340) von Läsionsindikationsinformationen des pathologischen Bildes auf der Grundlage von Merkmalsinformationen, die aus den mindestens zwei zweiten Instanzbildern extrahiert werden, umfasst:
Durchführen einer Merkmalskodierung an jedem zweiten Instanzbild, um zweite Merkmalsinformationen jedes zweiten Instanzbildes zu erhalten;
Durchführen einer Merkmalsfusion an den zweiten Merkmalsinformationen jedes zweiten Instanzbildes, um lokale Merkmalsinformationen des pathologischen Bildes für den Läsionskandidatenbereich zu erhalten;
Bestimmen einer zweiten vorhergesagten Wahrscheinlichkeit entsprechend jedem zweiten Instanzbild gemäß den lokalen Merkmalsinformationen und den zweiten Merkmalsinformationen jedes zweiten Instanzbildes, wobei sich die zweite vorhergesagte Wahrscheinlichkeit auf eine Wahrscheinlichkeit bezieht, dass das zweite Instanzbild den Läsionsbereich umfasst; und
Bestimmen von Läsionsindikationsinformationen des pathologischen Bildes auf der Grundlage einer Position des zweiten Instanzbildes, das der zweiten vorhergesagten Wahrscheinlichkeit entspricht, die eine zweite Bedingung in dem pathologischen Bild erfüllt.

2. Verfahren nach Anspruch 1, wobei das Durchführen einer Merkmalsfusion an den ersten Merkmalsinformationen jedes ersten Instanzbildes, um globale Merkmalsinformationen des pathologischen Bildes zu erhalten, umfasst:
Verarbeiten der ersten Merkmalsinformationen jedes ersten Instanzbildes durch einen Aufmerksamkeitsmechanismus, um ein Gewicht jedes Teils der ersten Merkmalsinformationen zu erhalten; und
Durchführen einer gewichteten Summierung über alle Teile der ersten Merkmalsinformationen gemäß dem Gewicht jedes Teils der ersten Merkmalsinformationen, um globale Merkmalsinformationen des pathologischen Bildes zu erhalten.

3. Verfahren nach Anspruch 1 oder 2, wobei das Bestimmen einer ersten vorhergesagten Wahrscheinlichkeit entsprechend jedem ersten Instanzbild gemäß den globalen Merkmalsinformationen und den ersten Merkmalsinformationen jedes ersten Instanzbildes umfasst:
Abbilden der globalen Merkmalsinformationen, um einen ersten Wahrscheinlichkeitskoeffizienten zu erzeugen, wobei sich der erste Wahrscheinlichkeitskoeffizient auf eine Wahrscheinlichkeit bezieht, dass das pathologische Bild den Läsionsbereich umfasst;
für jedes erste Instanzbild, Abbilden der ersten Merkmalsinformationen des ersten Instanzbildes, um einen zweiten Wahrscheinlichkeitskoeffizienten zu erzeugen, wobei sich der zweite Wahrscheinlichkeitskoeffizient auf eine Anfangswahrscheinlichkeit bezieht, dass das erste Instanzbild den Läsionsbereich umfasst; und
Bestimmen einer ersten vorhergesagten Wahrscheinlichkeit entsprechend dem ersten Instanzbild gemäß dem ersten Wahrscheinlichkeitskoeffizienten und dem zweiten Wahrscheinlichkeitskoeffizienten.

4. Verfahren nach einem der Ansprüche 1 bis 3, wobei das Bestimmen (340) von Läsionsindikationsinformationen des pathologischen Bildes auf der Grundlage von Merkmalsinformationen, die aus den mindestens zwei zweiten Instanzbildern extrahiert werden, umfasst:
Durchführen einer Merkmalskodierung an jedem zweiten Instanzbild, um zweite Merkmalsinformationen jedes zweiten Instanzbildes zu erhalten;
Durchführen einer Merkmalsfusion an den zweiten Merkmalsinformationen jedes zweiten Instanzbildes, um lokale Merkmalsinformationen des pathologischen Bildes für den Läsionskandidatenbereich zu erhalten; und
Bestimmen von Läsionswahrscheinlichkeitsverteilungsinformationen des pathologischen Bildes auf der Grundlage der lokalen Merkmalsinformationen und globaler Merkmalsinformationen des pathologischen Bildes, wobei die Läsionswahrscheinlichkeitsverteilungsinformationen eine Wahrscheinlichkeitsverteilung des Läsionsbereichs in dem pathologischen Bild angeben; wobei die Läsionsindikationsinformationen die Läsionswahrscheinlichkeitsverteilungsinformationen umfassen.

5. Verfahren nach Anspruch 4, wobei das Durchführen einer Merkmalsfusion an den zweiten Merkmalsinformationen jedes zweiten Instanzbildes, um lokale Merkmalsinformationen des pathologischen Bildes für den Läsionskandidatenbereich zu erhalten, umfasst:
Verarbeiten der zweiten Merkmalsinformationen jedes zweiten Instanzbildes durch einen Aufmerksamkeitsmechanismus, um ein Gewicht jedes Teils der zweiten Merkmalsinformationen zu erhalten; und
Durchführen einer gewichteten Summierung über alle Teile der zweiten Merkmalsinformationen gemäß dem Gewicht jedes Teils der zweiten Merkmalsinformationen, um die lokalen Merkmalsinformationen zu erhalten.

6. Verfahren nach Anspruch 1, wobei das Bestimmen einer zweiten vorhergesagten Wahrscheinlichkeit entsprechend jedem zweiten Instanzbild gemäß den lokalen Merkmalsinformationen und den zweiten Merkmalsinformationen jedes zweiten Instanzbildes umfasst:
Abbilden der lokalen Merkmalsinformationen, um einen dritten Wahrscheinlichkeitskoeffizienten zu erzeugen, wobei sich der dritte Wahrscheinlichkeitskoeffizient auf eine Wahrscheinlichkeit bezieht, dass der Läsionskandidatenbereich den Läsionsbereich umfasst;
für jedes zweite Instanzbild, Abbilden der zweiten Merkmalsinformationen des zweiten Instanzbildes, um einen vierten Wahrscheinlichkeitskoeffizienten zu erzeugen, wobei sich der vierte Wahrscheinlichkeitskoeffizient auf eine Anfangswahrscheinlichkeit bezieht, dass das zweite Instanzbild den Läsionsbereich umfasst; und
Bestimmen einer zweiten vorhergesagten Wahrscheinlichkeit entsprechend dem zweiten Instanzbild gemäß dem dritten Wahrscheinlichkeitskoeffizienten und dem vierten Wahrscheinlichkeitskoeffizienten.

7. Verfahren nach einem der Ansprüche 1 bis 6, wobei das Abtasten (310) eines pathologischen Bildes durch ein erstes Abtastschema, um mindestens zwei erste Instanzbilder zu erhalten, umfasst:
Partitionieren des pathologischen Bildes, um ein Hintergrundbild und ein Vordergrundbild in dem pathologischen Bild zu erhalten;
Segmentieren des pathologischen Bildes durch das erste Abtastschema, um mindestens zwei erste Kandidaten-Instanzbilder zu erhalten; und
Bestimmen eines ersten Kandidaten-Instanzbildes, das das Vordergrundbild umfasst, aus den mindestens zwei ersten Kandidaten-Instanzbildern als das erste Instanzbild.

8. Verfahren nach einem der Ansprüche 1 bis 7, wobei das Abtasten (330) des Läsionskandidatenbereichs durch ein zweites Abtastschema, um mindestens zwei zweite Instanzbilder zu erhalten, umfasst:
Extrahieren von Läsionskandidatenbildern aus dem pathologischen Bild gemäß dem Läsionskandidatenbereich;
Vergrößern des Läsionskandidatenbildes auf der Grundlage der Größe des pathologischen Bildes, um ein Zielläsionsbild zu erhalten, wobei eine Größe des Zielläsionsbildes mit einer Größe des pathologischen Bildes übereinstimmt; und
Abtasten des Zielläsionsbildes durch das zweite Abtastschema, um die mindestens zwei zweiten Instanzbilder zu erhalten.

9. Verfahren nach einem der Ansprüche 1 bis 8, wobei die Läsionsindikationsinformationen durch ein Läsionsbereichsbestimmungsmodell erhalten werden, wobei das Läsionsbereichsbestimmungsmodell ein Kodierungsnetzwerk, ein erstes Klassifikationsnetzwerk, ein zweites Klassifikationsnetzwerk und ein drittes Klassifikationsnetzwerk umfasst; wobei
das Kodierungsnetzwerk dazu konfiguriert ist, eine Merkmalskodierung an dem ersten Instanzbild und dem zweiten Instanzbild durchzuführen, um die ersten Merkmalsinformationen des ersten Instanzbildes und die zweiten Merkmalsinformationen des zweiten Instanzbildes zu erhalten;
das erste Klassifikationsnetzwerk dazu konfiguriert ist, die erste vorhergesagte Wahrscheinlichkeit entsprechend jedem ersten Instanzbild und die globalen Merkmalsinformationen des pathologischen Bildes gemäß den ersten Merkmalsinformationen jedes ersten Instanzbildes zu bestimmen;
das zweite Klassifikationsnetzwerk dazu konfiguriert ist, die zweite vorhergesagte Wahrscheinlichkeit jedes zweiten Instanzbildes und die lokalen Merkmalsinformationen des pathologischen Bildes für den Läsionskandidatenbereich gemäß den zweiten Merkmalsinformationen jedes zweiten Instanzbildes zu bestimmen; und
das dritte Klassifikationsnetzwerk dazu konfiguriert ist, die Läsionswahrscheinlichkeitsverteilungsinformationen des pathologischen Bildes gemäß den globalen Merkmalsinformationen und den lokalen Merkmalsinformationen zu bestimmen.

10. Verfahren zum Trainieren eines Läsionsbereichsbestimmungsmodells, wobei das Verfahren durch eine Computervorrichtung ausführbar ist und umfasst:
Erfassen (810) eines Trainingsprobensets, wobei das Trainingsprobenset mindestens ein pathologisches Probenbild umfasst;
Abtasten (820) des pathologischen Probenbildes durch ein erstes Abtastschema und ein zweites Abtastschema, um mindestens zwei erste Probeninstanzen und mindestens zwei zweite Probeninstanzen des pathologischen Probenbildes zu erhalten, wobei ein zweiter Überlappungsgrad, der eine zweite Bildüberlappung zwischen den zweiten Probeninstanzen angibt, größer ist als ein erster Überlappungsgrad, der eine erste Bildüberlappung zwischen den ersten Probeninstanzen angibt;
Bestimmen (830) von Läsionswahrscheinlichkeitsverteilungsinformationen des pathologischen Probenbildes auf der Grundlage von Merkmalsinformationen, die aus den mindestens zwei ersten Probeninstanzen extrahiert werden, und Merkmalsinformationen, die aus den mindestens zwei zweiten Probeninstanzen extrahiert werden, wobei die Läsionswahrscheinlichkeitsverteilungsinformationen eine Wahrscheinlichkeitsverteilung des Läsionsbereichs in dem pathologischen Probenbild angeben; und
Trainieren (840) des Läsionsbereichsbestimmungsmodells gemäß den Läsionswahrscheinlichkeitsverteilungsinformationen,
wobei das Läsionsbereichsbestimmungsmodell ein Kodierungsnetzwerk, ein erstes Klassifikationsnetzwerk, ein zweites Klassifikationsnetzwerk und ein drittes Klassifikationsnetzwerk umfasst; und
das Bestimmen (830) von Läsionswahrscheinlichkeitsverteilungsinformationen des pathologischen Probenbildes auf der Grundlage von Merkmalsinformationen, die aus den mindestens zwei ersten Probeninstanzen extrahiert werden, und Merkmalsinformationen, die aus den mindestens zwei zweiten Probeninstanzen extrahiert werden, umfasst:
Durchführen, durch das Kodierungsnetzwerk, einer Merkmalskodierung an jeder ersten Probeninstanz und jeder zweiten Probeninstanz, um die Merkmalsinformationen jeder ersten Probeninstanz und die Merkmalsinformationen jeder zweiten Probeninstanz zu erhalten;
Verarbeiten, durch das erste Klassifikationsnetzwerk, der Merkmalsinformationen jeder ersten Probeninstanz, um eine Pseudo-Etikette und eine erste vorhergesagte Wahrscheinlichkeit entsprechend jeder ersten Probeninstanz und globale Merkmalsinformationen des pathologischen Probenbildes zu erhalten; wobei sich die erste vorhergesagte Wahrscheinlichkeit auf eine Wahrscheinlichkeit bezieht, dass die erste Probeninstanz den Läsionsbereich umfasst;
Verarbeiten, durch das zweite Klassifikationsnetzwerk, der Merkmalsinformationen jeder zweiten Probeninstanz, um eine zweite vorhergesagte Wahrscheinlichkeit entsprechend jeder zweiten Probeninstanz und lokale Merkmalsinformationen des pathologischen Bildes zu erhalten; wobei sich die zweite vorhergesagte Wahrscheinlichkeit auf eine Wahrscheinlichkeit bezieht, dass die zweite Probeninstanz den Läsionsbereich umfasst; und
Verarbeiten, durch das dritte Klassifikationsnetzwerk, der globalen Merkmalsinformationen und der lokalen Merkmalsinformationen, um die Läsionswahrscheinlichkeitsverteilungsinformationen des pathologischen Probenbildes zu erhalten.

11. Computervorrichtung, umfassend einen Prozessor und einen Speicher, wobei der Speicher ein Computerprogramm speichert und das Computerprogramm durch den Prozessor geladen und ausgeführt wird, um das Verfahren zur Bestimmung eines Läsionsbereichs nach einem der Ansprüche 1 bis 9 oder das Verfahren zum Trainieren eines Läsionsbereichsbestimmungsmodells nach Anspruch 10 durchzuführen.

12. Computerlesbares Speichermedium, das ein Computerprogramm speichert, wobei das Computerprogramm durch einen Prozessor geladen und ausgeführt wird, um das Verfahren zur Bestimmung eines Läsionsbereichs nach einem der Ansprüche 1 bis 9 oder das Verfahren zum Trainieren eines Läsionsbereichsbestimmungsmodells nach Anspruch 10 durchzuführen.

## Revendications

1. Procédé de détermination d'une région de lésion, le procédé étant exécutable par un dispositif informatique, et comprenant :
l'échantillonnage (310) d'une image pathologique par un premier schéma d'échantillonnage pour obtenir au moins deux premières images d'instance, les premières images d'instance ayant un premier chevauchement d'image indiqué par un premier degré de chevauchement ;
la détermination (320) d'une région de lésion candidate dans l'image pathologique, sur la base d'informations de caractéristiques extraites des au moins deux premières images d'instance ;
l'échantillonnage (330) de la région de lésion candidate par un deuxième schéma d'échantillonnage pour obtenir au moins deux deuxièmes images d'instance, les deuxièmes images d'instance ayant un deuxième chevauchement d'image indiqué par un deuxième degré de chevauchement supérieur au premier degré de chevauchement ; et
la détermination (340) d'informations d'indication de lésion de l'image pathologique, sur la base d'informations de caractéristiques extraites des au moins deux deuxièmes images d'instance, les informations d'indication de lésion indiquant la région de lésion dans l'image pathologique, dans lequel la détermination (320) d'une région de lésion candidate dans l'image pathologique, sur la base d'informations de caractéristiques extraites des au moins deux premières images d'instance comprend :
la réalisation d'un codage de caractéristiques sur chaque première image d'instance pour obtenir des premières informations de caractéristiques de chaque première image d'instance ;
la réalisation d'une fusion de caractéristiques sur les premières informations de caractéristiques de chaque première image d'instance pour obtenir des informations de caractéristiques globales de l'image pathologique ;
la détermination d'une première probabilité prédite correspondant à chaque première image d'instance en fonction des informations de caractéristiques globales et des premières informations de caractéristiques de chaque première image d'instance, dans lequel la première probabilité prédite se réfère à une probabilité que la première image d'instance comprenne la région de lésion ; et
la détermination de la région de lésion candidate, sur la base d'une position de la première image d'instance correspondant à la première probabilité prédite qui satisfait une première condition dans l'image pathologique ;
dans lequel la détermination (340) d'informations d'indication de lésion de l'image pathologique, sur la base d'informations de caractéristiques extraites des au moins deux deuxièmes images d'instance comprend :
la réalisation d'un codage de caractéristiques sur chaque deuxième image d'instance pour obtenir des deuxièmes informations de caractéristiques de chaque deuxième image d'instance ;
la réalisation d'une fusion de caractéristiques sur les deuxièmes informations de caractéristiques de chaque deuxième image d'instance pour obtenir des informations de caractéristiques locales de l'image pathologique pour la région de lésion candidate ;
la détermination d'une deuxième probabilité prédite correspondant à chaque deuxième image d'instance en fonction des informations de caractéristiques locales et des deuxièmes informations de caractéristiques de chaque deuxième image d'instance, dans lequel la deuxième probabilité prédite se réfère à une probabilité que la deuxième image d'instance comprenne la région de lésion ; et
la détermination d'informations d'indication de lésion de l'image pathologique, sur la base d'une position de la deuxième image d'instance correspondant à la deuxième probabilité prédite qui satisfait une deuxième condition dans l'image pathologique.

2. Procédé selon la revendication 1, dans lequel la réalisation d'une fusion de caractéristiques sur les premières informations de caractéristiques de chaque première image d'instance pour obtenir des informations de caractéristiques globales de l'image pathologique comprend :
le traitement des premières informations de caractéristiques de chaque première image d'instance par un mécanisme d'attention pour obtenir un poids de chaque élément de premières informations de caractéristiques ; et
la réalisation d'une sommation pondérée sur tous les éléments de premières informations de caractéristiques en fonction du poids de chaque élément de premières informations de caractéristiques pour obtenir des informations de caractéristiques globales de l'image pathologique.

3. Procédé selon la revendication 1 ou 2, dans lequel la détermination d'une première probabilité prédite correspondant à chaque première image d'instance en fonction des informations de caractéristiques globales et des premières informations de caractéristiques de chaque première image d'instance comprend :
le mappage des informations de caractéristiques globales pour générer un premier coefficient de probabilité, dans lequel le premier coefficient de probabilité se réfère à une probabilité que l'image pathologique comprenne la région de lésion ;
pour chaque première image d'instance, le mappage des premières informations de caractéristiques de la première image d'instance pour générer un deuxième coefficient de probabilité, dans lequel le deuxième coefficient de probabilité se réfère à une probabilité initiale que la première image d'instance comprenne la région de lésion ; et
la détermination d'une première probabilité prédite correspondant à la première image d'instance en fonction du premier coefficient de probabilité et du deuxième coefficient de probabilité.

4. Procédé selon l'une quelconque des revendications 1 à 3, dans lequel la détermination (340) d'informations d'indication de lésion de l'image pathologique, sur la base d'informations de caractéristiques extraites des au moins deux deuxièmes images d'instance comprend :
la réalisation d'un codage de caractéristiques sur chaque deuxième image d'instance pour obtenir des deuxièmes informations de caractéristiques de chaque deuxième image d'instance ;
la réalisation d'une fusion de caractéristiques sur les deuxièmes informations de caractéristiques de chaque deuxième image d'instance pour obtenir des informations de caractéristiques locales de l'image pathologique pour la région de lésion candidate ; et
la détermination d'informations de distribution de probabilité de lésion de l'image pathologique, sur la base des informations de caractéristiques locales, et des informations de caractéristiques globales de l'image pathologique, les informations de distribution de probabilité de lésion indiquant une distribution de probabilité de la région de lésion dans l'image pathologique ; dans lequel les informations d'indication de lésion comprennent les informations de distribution de probabilité de lésion.

5. Procédé selon la revendication 4, dans lequel la réalisation d'une fusion de caractéristiques sur les deuxièmes informations de caractéristiques de chaque deuxième image d'instance pour obtenir des informations de caractéristiques locales de l'image pathologique pour la région de lésion candidate comprend :
le traitement des deuxièmes informations de caractéristiques de chaque deuxième image d'instance par un mécanisme d'attention pour obtenir un poids de chaque élément de deuxièmes informations de caractéristiques ; et
la réalisation d'une sommation pondérée sur tous les éléments de deuxièmes informations de caractéristiques en fonction du poids de chaque élément de deuxièmes informations de caractéristiques pour obtenir les informations de caractéristiques locales.

6. Procédé selon la revendication 1, dans lequel la détermination d'une deuxième probabilité prédite correspondant à chaque deuxième image d'instance en fonction des informations de caractéristiques locales et des deuxièmes informations de caractéristiques de chaque deuxième image d'instance comprend :
le mappage des informations de caractéristiques locales pour générer un troisième coefficient de probabilité, dans lequel le troisième coefficient de probabilité se réfère à une probabilité que la région de lésion candidate comprenne la région de lésion ;
pour chaque deuxième image d'instance, le mappage des deuxièmes informations de caractéristiques de la deuxième image d'instance pour générer un quatrième coefficient de probabilité, dans lequel le quatrième coefficient de probabilité se réfère à une probabilité initiale que la deuxième image d'instance comprenne la région de lésion ; et
la détermination d'une deuxième probabilité prédite correspondant à la deuxième image d'instance en fonction du troisième coefficient de probabilité et du quatrième coefficient de probabilité.

7. Procédé selon l'une quelconque des revendications 1 à 6, dans lequel l'échantillonnage (310) d'une image pathologique par un premier schéma d'échantillonnage pour obtenir au moins deux premières images d'instance comprend :
le partitionnement de l'image pathologique, pour obtenir une image d'arrière-plan et une image de premier plan dans l'image pathologique ;
la segmentation de l'image pathologique par le premier schéma d'échantillonnage pour obtenir au moins deux premières images d'instance candidates ; et
la détermination d'une première image d'instance candidate comprenant l'image de premier plan parmi les au moins deux premières images d'instance candidates en tant que première image d'instance.

8. Procédé selon l'une quelconque des revendications 1 à 7, dans lequel l'échantillonnage (330) de la région de lésion candidate par un deuxième schéma d'échantillonnage pour obtenir au moins deux deuxièmes images d'instance comprend :
l'extraction d'images de lésion candidates à partir de l'image pathologique en fonction de la région de lésion candidate ;
l'agrandissement de l'image de lésion candidate, sur la base de la taille de l'image pathologique pour obtenir une image de lésion cible, dans lequel une taille de l'image de lésion cible est cohérente avec une taille de l'image pathologique ; et
l'échantillonnage de l'image de lésion cible par le deuxième schéma d'échantillonnage pour obtenir les au moins deux deuxièmes images d'instance.

9. Procédé selon l'une quelconque des revendications 1 à 8, dans lequel les informations d'indication de lésion sont obtenues par un modèle de détermination de région de lésion, le modèle de détermination de région de lésion comprenant un réseau de codage, un premier réseau de classification, un deuxième réseau de classification et un troisième réseau de classification ; dans lequel
le réseau de codage est configuré pour réaliser un codage de caractéristiques sur la première image d'instance et la deuxième image d'instance pour obtenir les premières informations de caractéristiques de la première image d'instance et les deuxièmes informations de caractéristiques de la deuxième image d'instance ;
le premier réseau de classification est configuré pour déterminer la première probabilité prédite correspondant à chaque première image d'instance et les informations de caractéristiques globales de l'image pathologique en fonction des premières informations de caractéristiques de chaque première image d'instance ;
le deuxième réseau de classification est configuré pour déterminer la deuxième probabilité prédite de chaque deuxième image d'instance et les informations de caractéristiques locales de l'image pathologique pour la région de lésion candidate en fonction des deuxièmes informations de caractéristiques de chaque deuxième image d'instance ; et
le troisième réseau de classification est configuré pour déterminer les informations de distribution de probabilité de lésion de l'image pathologique en fonction des informations de caractéristiques globales et des informations de caractéristiques locales.

10. Procédé d'entraînement d'un modèle de détermination de région de lésion, le procédé étant exécutable par un dispositif informatique, et comprenant :
l'acquisition (810) d'un ensemble d'échantillons d'entraînement, l'ensemble d'échantillons d'entraînement comprenant au moins une image pathologique d'échantillon ;
l'échantillonnage (820) de l'image pathologique d'échantillon par un premier schéma d'échantillonnage et un deuxième schéma d'échantillonnage pour obtenir au moins deux premières instances d'échantillon et au moins deux deuxièmes instances d'échantillon de l'image pathologique d'échantillon, un deuxième degré de chevauchement indiquant un deuxième chevauchement d'image entre les deuxièmes instances d'échantillon étant supérieur à un premier degré de chevauchement indiquant un premier chevauchement d'image entre les premières instances d'échantillon ;
la détermination (830) d'informations de distribution de probabilité de lésion de l'image pathologique d'échantillon, sur la base d'informations de caractéristiques extraites des au moins deux premières instances d'échantillon et d'informations de caractéristiques extraites des au moins deux deuxièmes instances d'échantillon, les informations de distribution de probabilité de lésion indiquant une distribution de probabilité de la région de lésion dans l'image pathologique d'échantillon ; et
l'entraînement (840) du modèle de détermination de région de lésion en fonction des informations de distribution de probabilité de lésion,
dans lequel le modèle de détermination de région de lésion comprend un réseau de codage, un premier réseau de classification, un deuxième réseau de classification et un troisième réseau de classification ; et
la détermination (830) d'informations de distribution de probabilité de lésion de l'image pathologique d'échantillon, sur la base d'informations de caractéristiques extraites des au moins deux premières instances d'échantillon et d'informations de caractéristiques extraites des au moins deux deuxièmes instances d'échantillon comprend :
la réalisation, par le réseau de codage, d'un codage de caractéristiques sur chaque première instance d'échantillon et chaque deuxième instance d'échantillon pour obtenir les informations de caractéristiques de chaque première instance d'échantillon et les informations de caractéristiques de chaque deuxième instance d'échantillon ;
le traitement, par le premier réseau de classification, des informations de caractéristiques de chaque première instance d'échantillon pour obtenir une pseudo-étiquette et une première probabilité prédite correspondant à chaque première instance d'échantillon, et des informations de caractéristiques globales de l'image pathologique d'échantillon ; dans lequel la première probabilité prédite se réfère à une probabilité que la première instance d'échantillon comprenne la région de lésion ;
le traitement, par le deuxième réseau de classification, des informations de caractéristiques de chaque deuxième instance d'échantillon pour obtenir une deuxième probabilité prédite correspondant à chaque deuxième instance d'échantillon, et des informations de caractéristiques locales de l'image pathologique ; dans lequel la deuxième probabilité prédite se réfère à une probabilité que la deuxième instance d'échantillon comprenne la région de lésion ; et
le traitement, par le troisième réseau de classification, des informations de caractéristiques globales et des informations de caractéristiques locales pour obtenir les informations de distribution de probabilité de lésion de l'image pathologique d'échantillon.

11. Dispositif informatique, comprenant un processeur et une mémoire, la mémoire stockant un programme informatique, et le programme informatique étant chargé et exécuté par le processeur pour mettre en œuvre le procédé de détermination d'une région de lésion selon l'une quelconque des revendications 1 à 9, ou le procédé d'entraînement d'un modèle de détermination de région de lésion selon la revendication 10.

12. Support de stockage lisible par ordinateur stockant un programme informatique, le programme informatique étant chargé et exécuté par un processeur pour mettre en œuvre le procédé de détermination d'une région de lésion selon l'une quelconque des revendications 1 à 9, ou le procédé d'entraînement d'un modèle de détermination de région de lésion selon la revendication 10.
